# EUROPEAN PATENT APPLICATION

(11) **EP 4 628 146 A1**
(43) Date of publication of application: **08.10.2025**
(21) Application number: 24168866.2
(22) Date of filing: 05.04.2024
(51) Int. Cl.: A61M 39/18, A61M 39/10, A61M 39/24

(54) **BAYONET ASEPTIC CONNECTOR FOR FLUID CONNECTION AND A CONNECTION SYSTEM USING A PAIR OF SUCH CONNECTORS**

(71) Applicant: SARTORIUS STEDIM FMT SAS, 13400 Aubagne (FR)
(72) Inventor: Delasalle, Brice, 13400 AUBAGNE (FR); Blake, Florian, 13400 AUBAGNE (FR)
(74) Representative: Plasseraud IP

(57) **Abstract**

The connector (1) has a valve assembly with a tubular body (2), a plug (15) movable inside the body and a sleeve (20) to control plug position. A flange (3) on the body (2) delimits a recess where a latch (10) can interact with an inserting male part of a complementary connector or analogous structure. The connector comprises an aseptic attachment interface (5) at a front open end (2b) with a removable membrane (M). For aseptic fluid management in a connection involving two connectors, the connector (1) is configured to allow a disconnection after two successive steps, which are: moving the plug (15) in a closing position by a first manual actuation using the sleeve, and then displacing the latch by a second manual actuation using an external actuator (12) partly covering the flange and which is released by moving an blocker (14) in response to the first actuation.

## Description

### FIELD OF THE INVENTION

The instant disclosure generally relates to fluid management systems in the field of biopharmaceutical applications, including valve components for flexibility of fluid circulation, and more particularly to a fluid handling coupling or connector that allows a connection and disconnection, and to a connection/disconnection system using a pair of such connectors.

### TECHNICAL BACKGROUND

In the pharmaceutical, chemical field, that use tubing and assembling of components in the processing and transport for fluids (such as biopharmaceutical fluids), there is often a need for aseptic connection, at least for the first connection. Hoses carrying biopharmaceutical fluid may be commonly used for interconnecting flexibles pouches or similar parts of a circuit. The term "biopharmaceutical fluid", in the following, is understood to mean a product of biotechnology (culture media, cell cultures, buffer solutions, artificial nutrition liquids, blood products and blood product derivatives) or a pharmaceutical product or more generally a product intended for use in the medical field.

When using certain substances, it may be necessary to temporarily guarantee sufficiently sterile conditions after various components (pipes and/or containers) have been connected, while maintaining the possibility of uncoupling the connection when necessary. To this end, it is convenient to use fluid coupling components that can be unlocked by a specific action, and which may selectively block or allow flow of fluid through the coupling. Document US 2023/0122306 relates to fluid handling couplings that include each a manually openable and closable valve member, while being compatible for single-use aseptic fluid handling usage contexts. Connection is performed aseptically, using a pair of removable membranes.

Such kind of coupling has some drawbacks at the time of disconnection. Fluid at the connection interface is present and may flow as soon the operator separates the connectors.

More generally, there is still room for improvement regarding constructions of fluid connectors, in a way optimizing the way of operating with such fluid connectors, in aseptic conditions and/or in user-friendly conditions.

### OBJECTS AND SUMMARY

For improving situation, embodiments of the invention provide a connector for fluid connection, comprising a valve assembly that is provided with a tubular body, a plug movable inside the tubular body, and a sleeve to control position of the plug (plug position inside the tubular body, in an interior space), the connector comprising:
- a flange formed peripherally on the tubular body that extends around a longitudinal axis, the flange comprising a flange front face and a female connecting member with an axial access;
- a male connecting member including a first insert protruding axially along a protruding direction , the first insert including a fastening region;
- an aseptic attachment structure including a connector front face and a membrane removably attached to the connector front face to seal an opening of the tubular body (which is the opening for fluid passage inside the tubular body or any suitable interior space), the connector front face possibly including the flange front face;
- a housing or flange peripheral region extending around the interior space at a rear of the flange front face (typically at a rear flange face with a rear access, the housing or peripheral region preferably delimiting a recess opening rearwardly), the axial access forming an access (front insert access) to the housing or peripheral region and allowing insertion of a second insert of same structure as the first insert, the second insert including a fastening region, the second insert being formed in a complementary connector that is suitable to be coupled to said connector for enabling the fluid connection;
- unlocking the locking configuration of the second insert
- latching means for instance a latch provided with slidable rigid portions or rigid frame, arranged inside the housing or at the peripheral region), the latching means being configured to allow the second insert, arranged in the flange peripheral region, to be axially retained in a locking configuration of the fluid connection by an abutment at the fastening region of the second insert, the locking configuration being obtained when the second insert is inserted in the female connecting member;
wherein the connector has movable latching means and/or is configured to allow a disconnection, by releasing the fastening region of the second insert from the latching means, after successively:
- moving the plug in a closing position by a first manual actuation using the sleeve; and
- unlocking/disengaging the insert (to leave the locking configuration of the insert), possibly by displacing one amongst the latching means and the fastening region of the second insert, by a second manual actuation using an external actuator mounted on the flange, preferably covering the flange (externally), the second external actuator being unblocked due to the first actuation with the sleeve moving an actuation blocker preventing the second manual actuation (such action of the sleeve being performed at/being a part of the first actuation).

Such kind of connector thus combine ability for aseptic connection, using the membrane of the aseptic attachment structure to initially release the opening/front opening once the mechanical coupling is obtained (with each insert received in a corresponding connection axial access), and ability to reclose this opening with combined effects that are required for the mechanical disconnection. The sleeve may be thus considered as a first unlocking part to be actuated for simultaneously driving the plug and the actuation blocker, while the external actuator is a second unlocking part. It is understood the external actuator, for instance pushable when released, is always blocked away from the latching means during operations/fluid management, until the actuation blocker is driven/displaced to a release configuration, thanks to the sleeve.

In some embodiments, the sleeve is an outer sleeve, possibly of circular section, carried around a cylindrical portion of the tubular portion, at the rear of the flange. The sleeve may be positioned in an axial area that is shifted/offset or spaced rearwardly relative to a recess of the housing, where the latching means are received. In some variants, all or part of the sleeve may be arranged inside a part of the tubular body and a handle or similar actuating portion may allow a manual rotation of the sleeve. Of course, any suitable actuating operation causing the plug to be moved, preferably linearly along direction of the longitudinal axis of the tubular body, can be adopted.

The plug may be longitudinally shorter than the tubular body and concealed/hidden inside the tubular body, while reaching the front opening in the closing position. Typically, the plug may be movable in interior space delimited by the tubular body and has a sealing position inside the interior space when reaching the closing position. Optionally, the closing end portion of the plug is radially in contact with an inner edge of the tubular portion that is provided to delimit the opening/front opening. An annular sealing element may be provided on this closing end portion, circumferentially. The closing end portion of the plug may also be axially in contact with an opening rim, either with an inner shoulder or an annular rim portion (beveled edge for instance) facing rearwardly. This annular axial contact may be obtained in an annular margin portion of the closing end portion.
The annular margin portion may be thinner than a remainder of the closing end portion. A front surface of the closing end portion may be planar.

The flange may have two opposite outer sides that are parallel sides, possibly extending substantially rectilinear. The external actuator can be mounted slidably, covering the two opposite outer sides that guide two parallel arms of the external actuator. A pushing surface may be provided in a piece or assembly forming the external actuator, with the pushing surface extending transvers to the two parallel arms.
Whatever the shape of the external actuator and the number of piece(s) involved, the external actuator may be operatively coupled to the latching means so that, when the connector is coupled to another connector (analogous connector or identical connector with an interlocking at a first insert of the connector and at a second insert of the other connector, using the female connecting members and associated latches/latching means), a displacement of the external actuator causes release of the engagement between the latching means and the second insert, whereby the external actuator can unlock the fluid connection.

According to embodiments, one or more of the following features may be provided:
- the tubular body extends around the longitudinal axis between a first open end (forming rear end when considering the longitudinal axis extends along a rear-front direction) and a second open end.
- the tubular body is a single piece, including the flange.
- the tubular body is an assembly, including the flange and a tubular extension part (optionally forming a nozzle at a rear end), the tubular extension part being inserted inside or around a tubular fitting integral with the flange.
- the aseptic attachment structure includes the second open end, the membrane being removably attached to the flange front face or the tubular body to seal the opening provided at the second open end. - the flange is integral with the tubular body and defines a flange outer circumference.
- the housing or peripheral region is delimited by an external side wall of the flange;
- the flange has an external side wall having a longitudinal extension;
- the flange has an external side wall that is locally provided, preferably in a corner of the flange, with a side wall opening so that an abutment part of the external actuator protrudes radially inward and/or engage the latching means through the side wall opening.
- the external side wall is forming an outer frame surrounding a recess, with the latching means housed in an interior volume of the outer frame, possibly of rectangular shape.
- an interior space of the tubular body is accessible via the opening;
- the tubular body is carrying a seal in a front groove interposed between the second open end and a flange front projection.
- an annular recess housing the latching means is delimited between the flange front projection and the external side wall.

In some options, the actuation blocker is configured to prevent the displacement in a first actuation blocker position, which is a position in a first angular sector around the tubular body. The actuation blocker may be a partial collar, a rounded rim or similar radially protruding relief arranged on an outer surface of the sleeve, typically at a sleeve end that is proximal to the flange. For preventing the displacement of the external actuator, the actuation blocker may face an inner bulging part of the external actuator, causing a contact or an early contact that prevent sufficient sliding or suitable movement to drive the latching means toward a release configuration.

The plug may be in contact/engaged with the sleeve, using plug part that extends radially across the tubular portion. In some options, the plug is coupled to the sleeve and guided by the tubular body, the sleeve extending around the tubular body.
The sleeve and at least one protruding part protruding radially outward, provided on the plug, may cooperate in a bayonet connection system.

For preventing accidental release of the latch/latching means, a return effect provided by a resilient portion (of the latching means or of a spring biasing the latching means) may be involved for maintaining an engaged position of the latching means with respect to the second insert (of the complementary connector in a connection state) that is
- inserted through the axial access; and
- arranged to protrude inside the housing (at the flange side opposite from the connection side).

The actuation blocker may protrude radially outward from a cylindrical surface of the tubular body, the actuation blocker extending transversely or perpendicular to protruding direction of the second insert. The contacting part of the latching means, in radial contact or any suitable contact with the second insert to prevent extraction of the second insert (insert of the other/complementary connector). Any suitable structure for the actuation blocker may be provided, possibly with the actuation blocker protruding in radial direction along same or similar direction of a return force applied by the latching means for maintaining the second insert.

The sleeve may a rotating sleeve with the actuation blocker integral with the sleeve, possibly included in same plastic piece. As a result, as long as the actuation blocker is in the first position for cooperating with an abutment member of the external actuator (the abutment member selectively occupying a first angular sector in this first position), the latching means and the actuation blocker co-act to maintain the second insert anchored by suppressing any movement along a radial direction that can be a pushing direction exerted on the external actuator. By rotating the sleeve to control the plug and move the plug to reach the closing position inside the tubular body, the actuation blocker is rotated as well and may occupy a different angular sector not matching with and typically fully distinct from the first angular sector (thus facing a second angular section of the external actuator where there is no abutment member preventing a pushing action along the pushing direction).

More generally, it is understood the actuation blocker can have a second actuation blocker position upon actuation of the sleeve. The second actuation blocker position, which is obtained in response to an actuation of the sleeve, preferably without any longitudinal movement of the sleeve, is a position allowing the displacement of the external actuator.
In some embodiments, in a second position of the actuation blocker, the actuation blocker can be positioned in a second angular sector around the tubular body, typically facing a hollow/empty space provided in the external actuator.

The insert may be a single protruding member of the connector, which protrudes from the front face with a protruding extension greater than a depth (or longitudinal extension) of the axial access provided by the female connecting member. A complementary of shape/circumference may be used for having the insert filling the axial access.
In some variants, the insert is part of a group of parallel inserts that either engage into a single opening or slot forming the axial access, or engage in different openings available on the flange (and each opening at the flange front face).

The latching means may cooperate and engage with an insert end that protrudes beyond an opening of the axial access, so that the insert end is housed in a recess of the housing. The recess, possibly an annular recess, can be wider and/or of larger section than section of the axial access filled with the insert.
The insert may be an inserting tab, possibly provided with a curved (slightly curved for instance) profile.
The fastening region may include an outer groove (facing radially outward), compatible with a U-section of a latch, with the locked position of the latch corresponding to an engagement of the base of the U-section inside the outer groove, possibly with a return-effect or similar constraint used to maintain the locked position. The latch may be movable, for instance slidable without any rotation. The latch may be actuatable like a guillotine except the latch contact edge may be concave to follow shape of curved outer groove formed on the inserting tab.

The flange may extend perpendicular to the longitudinal axis of the tubular body. An annular seal may be received in the flange front face, being flush with an annular rim delimiting the front opening (opening at the second end) of the tubular body. The flange may be molded from plastic material, for instance thermoplastic, and can include an intermediate annular wall interposed (with a concentrical arrangement) between an inner flange side wall and the external side wall. Such configuration may provide a compact flange with:
- a first groove of annular shape for the seal (at the front side), arranged between the intermediate annular wall and the inner flange side wall that includes an end of the cylindrical inner face of the tubular body;
- and a second groove of annular shape, with opposite orientation as compared to the first groove, defining the annular recess for receiving the latching means and the insert end engaged by the latching means.
The second groove may be delimited between the external side wall and the intermediate annular wall

According to embodiments of the valve assembly, one or more of the following features are implemented:
- the sleeve surrounds the plug.
- each of the sleeve and the plug are in contact with a same piece forming the tubular body.
- the plug is movable between a distal position away from the second open end and allowing a fluid to flow through the second open end, and a proximal position in which the second open end is engaged by the plug to seal the opening;
- in a proximal position of the plug, preferably obtained when a lug or similar protruding part of the plug extends at an end of a guiding track provided by the sleeve, an aseptic disconnection of the connector is allowed, typically due to release of an actuator adapted to control a position or configuration of the latching means.
- the end of the guiding track is apparent to show that the closing position of the plug as reflected by the position of the lug, engaged at this end of the guiding track.
- the end of the guiding track is proximal to the flange, and set at a distance from another guiding track end that may be proximal to the first end of the tubular body and distal from the flange.
- in the proximal position of the plug, the second open end is engaged by the plug to seal the opening.
- the plug has a closing member provided at a front plug end, the front plug end being surrounded by the second open end of the tubular body.
- an annular seal surrounds the front plug end, the annular seal being preferably located in an annular groove.
- the annular groove for the seal has a longitudinal extension greater than longitudinal extension of the annular recess receiving the latching means.

According to some embodiments, the sleeve has a first position, in which the actuation blocker is facing an abutment part of the external actuator and prevents the displacement (inward radial displacement, for instance) of the external actuator.
The sleeve may be rotatably movable to occupy a second position in which the actuation blocker is offset relative to the abutment part. Optionally, the second actuation blocker position can be selectively obtained for the second position of the sleeve. In some cases, an additional action is required, using an auxiliary lock for instance. Whatever, the way a connection using the connector can be locked, the sleeve may be selectively arranged in the first position to maintain the plug in the distal position, so that unlocking of the fluid connection by the displacement of the external actuator previously requires moving the sleeve in the second position.
The external actuator may be an outer disconnection button that releases a locking slider (optionally a ring-shaped locking slider) or similar latching means adapted to interact with the insert received axially through the flange. A leaf spring or push-push actioner may be used in a variant.

According to a first option, the actuation blocker is a part of the sleeve and is arranged radially between the tubular body and the external actuator, at least in the first position of the sleeve.
According to a second option, the actuation blocker is distinct from the sleeve and is arranged radially between the tubular body and the external actuator, at least in the first position of the sleeve.
The sleeve may have a annular cross-section that is circular, the actuation blocker being a radial bulge, a partial or irregular collar or any suitable protrusion carried by a cylindrical portion of the sleeve.

The sleeve may be only rotatable, without any longitudinal displacement. Alternatively, the sleeve may be longitudinally pushable to drive the plug linearly, with an ability to have the sleeve then rotated by a twisting action, to displace (out of the way of the external actuator) the actuation blocker, which may be a small tab preventing depression of the external button-like actuator.
Possibly, the plug is connected to a cup-like sleeve to follow the longitudinal movement of the sleeve without moving in rotation. The longitudinal driving may be obtained by sandwiching at least one linking arm, rotatable with the sleeve, with each linking arm maintained axially fixed in position relative to the plug, for instance being maintained between two axial abutments that occupy an angular sector sufficient to allow a limited rotation or twisting of the linking arm. A guiding track along the tubular body may be present to guide the stroke of the sleeve.

In some options, the sleeve can be rotated to open and/or close the plug acting as a valve member, preferably with the plug couplet to anti-rotation means. The sleeve may be rotatably mounted around a portion of the tubular body that is provided with a linear (rectilinear or instance) guide for guiding the plug longitudinally and prevent plug rotation. In some embodiments, the sleeve may include a projection extending radially inward, engaging a rib or groove provided on the plug, preferably at a rear portion of the plug, for slidably move the plug toward the flange front face and reach the closing position of the plug.
The sleeve, manually operable, may have a rear end, at the opposite from the flange, which is closer from the first end of the tubular body than the closing plug end in any of the positions of the plug. The rear end of the sleeve may include a collar, forming a gripping collar.

Embodiments of the mechanical coupling part of the connector may include one or more of the following particulars:
- the male connecting member is arranged to protrude axially forward, preferably forward relative to a flange front face.
- the male connecting member is arranged to protrude axially forward to form a free end of the connector,
- the flange comprises the male connecting member (formed as a protrusion in the flange, for instance), the first insert protruding axially from the flange front face provided with the axial access.
- the male connecting member and the female connecting member are distributed on the flange front face, so that the connector is genderless.
- two identical connectors are adapted to be fastened, with each insert radially offset and protruding parallel to the longitudinal axis.
- each axial access of the female connecting member allows insertion along a direction parallel to the longitudinal axis.
- the insert comprises an inserting tab, possibly a flat inserting tab.
- the insert or inserting tab is shifted/offset on a first side relative to the longitudinal axis, a membrane of the aseptic attachment structure being elongated from a covering portion covering the opening (front opening) to a membrane pulling portion.
- the insert or inserting tab is offset laterally relative to the membrane.
- the latching means are radially movable, preferably toward the longitudinal axis of the tubular body, to engage the fastening region of the insert protruding end that protrudes beyond a transverse flange wall that surrounds an annular seal carried by the flange around the opening (central opening at the front of the connector).
- the insert fastening region may be facing radially outward when forming of a groove or similar relief part, to be engaged by a part of the latching means that is movable along a radial direction.
- the insert, which may have a beveled free edge or a slanted/thinned edge, may slide inside the axial access of the female connecting member until pushing radially outward the latch or latching means that are movable in the housing or recess provided peripherally in the flange.
- a locking is obtained when a blocking bar of the latching means is engaged between two reliefs of the insert or inside a groove of the insert.

A position-locking system is obtained when mating the insert of the male connecting member (provided in another connector) with the female connecting member of the connector, combined with the latching means to have an anti-return effect once the insert is fully/properly inserted. With such connectors being of the genderless type, a pair of latching means distributed in the two connectors can be combined with the female connecting member distributed in the two connectors, so that latching means - insert engagement at a rear of first connector flange is simultaneously obtained with a latching means - insert engagement at a rear of the second connector flange forming the fluid connection together with the first connector. The front connection between the connectors may be allowed while hoses or tubes are connected at the respective rear parts, which may be provided with a nozzle or barbed end suitable for fluid connection with a flexible hose/tube.
Of course, the connection can be obtained only when the flanges of the connectors are mutually facing each other, with a parallel configuration allowing the respective inserts to be displaced parallel in opposite directions, until a locking action.

According to a particular feature, the inserting tab, which protrudes from a flange face (typically provided with a mouth of the opening/front opening) is offset on a first side relative to the opening, while the female connecting member is offset on a second side, opposite to the first side, relative to the opening. Typically, a base of the inserting tab, the opening and the axial access of the female connecting member may be arranged in alignment (transverse alignment) along a transverse direction perpendicular to the longitudinal axis. Optionally, the membrane may extend/be elongated, also transversely, along a direction perpendicular to this alignment direction (transverse alignment). With such distribution, a fluid connection using the connector and another identical connector (which are directly coupled at the coupling parts thereof), may be obtained with the inserts serving as visual indicators for the suitable alignment between the male and female complementary parts allowing the fastening. The locking may be initiated only at the end of the respective insertions, for instance with the fastening region in each insert provided at a distance of less than 8 mm, preferably less than 5 mm, from an insert free edge.

According to options for obtaining a tight closing of the front opening, which is typically a central opening centrally crossed by the longitudinal axis of the tubular body, the plug may comprise one or more of the following features:
- the plug is provided with a cam follower, possibly forming the lug or protruding part formed on a side wall of the plug.
- the plug is provided with a cross portion that interconnects a tubular rear portion of the plug, adapted to slide along an inner face of the tubular body, and a plug front end that includes a closing portion, possibly formed as a generally radial portion.
- a fluid interior passage delimited by the tubular rear portion axially opens in a partitioned chamber of the interior space, which is partitioned (possibly divided in four fluid passage sections) by the cross portion arranged as a partitioning wall having a X-profile.
- the plug is made of a single piece, preferably of plastic material, possibly completed with at least one annular sealing member that surrounds the plug closing end.

According to some options for controlling the plug, one or more of the following features may be provided:
- two lugs, arranged as radial protrusions, are provided for allowing a driving of the plug by the sleeve or any suitable driving means available at a rear side of the flange.
- a bayonet connection is obtained, with the lugs being prevented from rotating around the longitudinal axis, preferably using a rectilinear guide, such as a rectilinear slot portion, on a rear cylindrical portion of the tubular body.
- each rectilinear slot portion is part of a slot that opens rearwardly, the slot being formed in a cylindrical wall to have each lug entirely crossing radially the cylindrical wall (with a rear access, mounting of the plug inside the tubular body, from a rear open end of the tubular body, is facilitated).
- the sleeve comprises a guiding track (possibly distributed with two grooves) to guide movement of the at least one protruding part (possibly including two lugs respectively associated to the grooves).
- the at least one protruding part may be engaged with retaining means when the plug is in a closing position with the at least one protruding part positioned at an end of the guiding track.
- a retaining element, formed in the sleeve, can be arranged at the end of the guiding track, which is preferably a guiding track end that is proximal relative to the flange.
- the retaining element may be a protrusion that is tapering towards a free end, forming a tooth for instance.
- the plug is in the proximal position when the at least one protruding part is engaging with the retaining element, preferably with the lugs or protruding parts arranged at a rear of the flange.

With retaining means in the guiding track, for instance two retaining elements provided at groove front ends, end of the stroke of the sleeve corresponds to a point of non-return, with the connector thus being in a closed state (the plug has already reached the closed position due to advance of the plug forward). A rotational actuation of the sleeve may be of interest, to rotationally lock the closed position of the plug. The closed position can be a definitive (final) position and the connector will never be able to move back and re-open the fluid passage.

Each protruding part or lug may act as a slidable detent element, slidable along a slot (slot of the guiding track) provided in the tubular body. Optionally, the slot (distinct from the bayonet slot(s) possibly forming the guiding track) may be a rectilinear slot that is adjacent to the first open end of the tubular body, provided that the sleeve can rotate (typically without any longitudinal displacement), typically around the tubular body.

The sleeve or similar actuating portion, manually operable, may be coupled to the valve to form a driving mechanism part. The driving mechanism part may be coupled before being mounted on a shaft formed by the rear end of the tubular body, so that:
- the inner part of the driving mechanism part, which includes the plug, can move longitudinally in the interior space, possibly as far as the front end opening.
- the external part of the driving mechanism part, which includes the sleeve, is rotatable to change angular sector of the actuation blocker.

The external actuator may be pushed or similarly actuated. The external actuator may be pushed along a direction which is parallel to an extension of the membrane, possibly identical to a direction for pulling the membrane when establishing a fluid connection using the two connectors.
Whatever the kind of actuation, the external actuator can cover three distinct sides of the flange and may be slidably mounted relative to the flange. In options, the external actuator comprises two sliding arms between which the female connecting member and the male connecting member extend (typically with the female connecting member interposed between the central front opening and a first arm, while the female connecting member is interposed between the central front opening and a second arm opposite to the first arm).

The latching means may comprise one or more pieces, for instance a single plastic piece that has a ring structure or a plate structure with a wide central opening. The latching means or latch may include a locking slider of generally annular shape, with two opposite parallel sides and a central opening through which a rear cylindrical portion of the tubular body and an insert (typically the insert of the male connecting member from a complementary connector mating with the connector) can pass.
The locking slider may be an unlockable locking element, provided with an outer part, possibly annular with two straight sliding sides. The locking slider may have a slot at an end that corresponds to a pushing side of the external actuator, the slot separating two flexible tabs. More generally, a deformable portion can be provided, preferably axially offset relative to a contact portion driven by the external actuator (the contact portion also extending, transversely, at the same end of the locking slider).

The locking slider, housed in an annular recess of the housing, may also comprise a bridge portion or similar inner actuation portion interconnecting the two side members forming the two parallel sides, with the bridge portion suitable to be pushed, following pushing movement of the external actuator. Such inner actuation portion, located in the recess, can be configured to be pushed toward the longitudinal axis in order to unlock the connection, with a retaining portion of the locking slider (that is part of the locking plate) opposite the inner actuation portion. Of course, in a by-default configuration with the external actuator maintained away from the locking slider, the locking slider may be in a first position adapted to abut against the fastening region of the insert (second insert) passing through the axial access (fastening region, which may include a retaining external annular rim of the second insert) to prevent removal of the male connecting member of the complementary connector.

According to embodiments, the locking slider may slide linearly, parallel to the first transverse direction. In some embodiment of the connector, one or more of the following features are provided:
- the housing can form a slide extending generally within a transverse plane perpendicular to the longitudinal axis.
- the latching means comprise a locking slider that may be flat and that engages the second insert at the opposite or away from a contact area between the external actuator and the locking slider.
- the latching means comprise a locking slider distinct from the tubular body and inserted in the housing (or recess at the rear of the flange), the locking slider being configured to perform the retaining of the second insert at the fastening region of the second insert.
- the external actuator can be pushed along a first direction (possibly the first transverse direction) and the locking slider is configured to provide an elastic return force directed toward another direction, which is a second direction (possibly the second transverse direction) to radially engage the insert.
- the second direction can be substantially perpendicular to the first direction.
- the external actuator is provided with an abutment member, protruding inwardly.
- a slanted surface (which can mean a beveled surface) is provided in at least one amongst the abutment member and the locking slider, the slanted surface being configured so that movement of the external actuator along the first direction causes the locking slider to be driven along an unlocking direction that is opposite to the second direction.

In some embodiments, the housing forming the recess is provided with the external side wall (of the flange) so that the recess is of annular shape for receiving the latching means. Typically, one or more of the following features may be provided for the fluid connector:
- the external actuator, which is pushable along a first direction, is provided with an abutment member to drive said one amongst the latching means and the insert/fastening region of the insert, via an opening provided in the external side wall (18).
- the abutment member is adjacent to the abutment part (included in the external actuator) and/or radially protrudes inside the housing at a corner portion of the housing.
- the external actuator is C-shaped or U-shaped, as observed in a front view of the connector.
- the arms of the external actuator have fixation members cooperating with complementary members provided on an external side wall of the flange (which typically delimits the recess housing the latching means/latch).

The mounting of the plug can be performed from a rear end of the tubular body. More generally, embodiments of the valve assembly may be obtained with one or more of the following features:
- the valve assembly is springless.
- the sleeve comprises at least one cam slot, in which one or more radial protrusions (lugs for instance) of the plug engage.
- the tubular body has a longitudinal slot, preferably rectilinear, interposed between the plug and the sleeve.
- the one or more radial protrusions are protruding radially (outward) through the longitudinal slot.
- the tubular body supports or includes a tubular end part that retains the sleeve in an axial position between an annular abutment of the tubular end part and a shoulder portion.
- the shoulder portion may delimit, with the external side wall, an annular recess for housing the latching means.
- the tubular end part may comprise a nozzle with or without barb(s), preferably forming a barbed end for a flexible hose or tube.

According to another aspect, embodiments provide a connection system comprising a first connector and a second connector (possibly identical connectors), typically interlocked using the male and female connecting members distributed in the two connectors (which may be genderless connectors),
wherein the connectors are each a connector for fluid connection as above defined, a difference in angular position between the first connector and the second connector being 180° in an operating mode of the connection system with the respective flanges of the first and second connector being facing mutually, with an annular contact provided between two annular seals, each of the two annular seals being carried in the corresponding connector on a flange face that is opposite to a recess of the housing,
wherein for each of the connectors, the latching means extend (possibly by being slidably mounted) in the connector recess, and wherein, when the plugs are in their sealing position, the external actuator in the first connector, on the one hand, and the external actuator in the second connector, on the other hand, are simultaneously pushable along a same direction, due to a longitudinally extending interconnection linking the external connector of the first connector to the external connector of the second connector.

With such arrangement, if only one of the plugs is not moved in its closing position, it is sufficient to block both external actuators. This may prevent a fluid leakage when the plug is positioned at a front closing position, with a closing portion of the plug possibly being adjacent or reaching a junction plane where the openings of the connectors mutually join. More generally, blocking an external actuator by an actuation blocker automatically means blocking the other external actuator, due to the securing link. Risk of unintentional disconnection is also prevented or cannot result in consequences for the parts that are separated from the disconnection area. Automatic correlation between the closing position of the plug and the release position of the actuation blocker may be allowed with any spring or similar elastic return member present in the fluid passage.

The connectors, when chosen to be of the genderless type, simplify the operations as there is no risk of confusion between different connectors. The structure of each connector may be flexible. The sleeve may be a rotating sleeve or it may be a slidable component that moves the plug in response to a longitudinal actuation. The sleeve may rotate along a rotation axis that is parallel to a central or longitudinal axis of the tubular body, with the sleeve possibly concentrically arranged around a portion of the tubular body that protrudes rearwardly from a flange rear face (with the flange front face being a connection face, initially covered by the membrane in the aseptic attachment structure).

The connection system may have the external actuators already linked before any removal of membrane sealing a corresponding a central front opening. In some embodiments, the external actuator is provided with one or more of the following features:
- the external actuator comprises engagement parts, including an insert member or pin, for having a pair of external actuators acting simultaneously when performing disconnection of the fluid connection obtained by the connection system (typically using the connector and the complementary connector that is of identical structure).
- the external actuator of the complementary connector can be identical to the external actuator of the connector in the connection system.
- the external actuator of the complementary connector can be arranged at a same angular sector (as the other external actuator) in a mutually engaged state of the external actuators of said pair with the engagement parts interlocked.
- displacement of the external actuator provided in the connector is synchronized with displacement of the other external actuator provided in the complementary connector.
- the external actuator displacement is allowed selectively when each plug (in the respective connectors) is arranged in the proximal position (plugging position to seal the front opening at the second open end of the tubular body).

According to another aspect, a method is provided to obtain a connector simple of use and efficient for keeping control of the aseptic operations, when establishing a fluid connection of such connector.

The method of assembling a fluid connector comprises:
- inserting a plug inside a tubular body via a first open end of the tubular body, which is a tubular body rear end;
- forming an aseptic attachment structure with a flange formed peripherally on the tubular body, in order to form a flange front face partly covered by the membrane while a male connecting member offset relative to the membrane and arranged integral with the tubular body is protruding frontward beyond the membrane, the flange being provided with a female connecting member having an axial access (and preferably with the male connecting member that protrudes from the flange front face);
- housing a latch or a retaining surface in a recess formed by the flange, the axial access axially opening in the recess;
- mounting an external actuator on the flange, so that the external actuator can be pushed;
- engaging the latch or the retaining surface in the recess to provide a return-effect allowing the latch or the retaining surface to interfere with a crossing insert of another connector, which is an insert of same length and shape as an insert provided in the male connecting member, the crossing insert passing through the axial access and protruding in the recess to be automatically engaged by the latch or the retaining surface;
- placing an actuation blocker in an interposed position between the pushing surface and the sleeve, which is a blocking position so that the actuation blocker prevents external actuator to be moved, for instance moved radially inward, when pushed along the first transverse direction;
   whereby the latch or the retaining surface can retain the fitting insert, in a locking configuration established by interconnecting the connector with another identical connector,
   wherein in mounted state, the external actuator is actuatable and movable to have an engaged state, in which the external actuator engages one amongst:
- the latch or the retaining surface,
- and the fitting insert,
   the engaged state being obtained in a pushed state of the external actuator to unlock the retaining of the fitting insert, provided the actuation blocker has been previously removed from the blocking position;
   and wherein the sleeve is configured to be manually operable to simultaneously:
- move the plug to reach the tubular body front end in a closing position sealing the central opening, in absence of the membrane; and
- remove the actuation blocker from the blocking position.

In some embodiments, the membrane extends radially, preferably beyond the flange to provide a pull portion, along a first transverse direction that is perpendicular to a longitudinal axis of the tubular body passing through the central opening. The female connecting member and the male connecting member may be distributed on either sides relative to the membrane. The mounting of the external actuator on the flange can be performed so that the external actuator is suitable to be pushed along the first transverse direction and/or pushed from a pushing surface of the external actuator which extends along a second transverse direction that is transverse or perpendicular to the first transverse direction.

For installing the sleeve, a guiding portion of the tubular body can form a mounting shaft allowing rotation of the sleeve, preferably by preventing rotation of the plug. In preferred options, a bayonet connection can be obtained to make operation of the sleeve user-friendly. In some variants, rotation of the sleeve for the removal (which may be a lateral shifting) of the the actuation blocker from the blocking position can be obtained after a preliminary translation, already allowing the plug to be adjacent to or reach a final closing position. A relatively slight pinching pressure on each sleeve (which may have a slot or a cup-shape in some options) may be sufficient to reach a provisional position where the actuation blocker becomes rotatably engaged with the sleeve or becomes properly positioned for starting the removal by a rotational shifting.

According to another aspect, it is provided a use of two connectors for fluid connection that are each forming a connector as defined above, wherein the two connectors, typically genderless, are mutually fastened at the flanges that are facing one each other with an interspace, each flange carrying the external actuator that is movable relative to said flange) to allow a release of a retaining contact at the latching means,
and wherein each connector has the external actuator comprising a front portion that is:
   - partly covering the flange (which is the flange supporting the external actuator);
   - facing in a direction away from the sleeve; and
   - provided with a linking member or pin that protrudes from the front portion;
so that in a connection system obtained by coupling the two connectors, a link is obtained by the linking members or pins so that a disconnection of the fluid connection system is only available when having:
   - each actuation blocker of the respective connectors occupying the second actuation blocker position, which means each plug is in the sealing position in the two connectors.

The fluid connection is obtained with symmetrically opposed plugs displaced as opposed pistons which can optionally contact one each other when the two plugs are in the closing position. The connectors are compliant with a simple way of establishing a fluid connection, while securing a synchronized unlocking of the interlocked parts (female and male connecting members), which can thus be pulled and separated in an easy way: an operator may have a left thumb pushing on the first external actuator, a right thumb pushing on the second external actuator, so that a separation of the connectors taken each in one of operator's hands is:
- easily obtained, without any tool; and
- selectively obtained for closed state of the connectors, typically without dead leg or similar lost interspace between the plugs at the time of performing the disconnection.

### BRIEF DESCRIPTION OF THE DRAWINGS

The figures of the drawings are now briefly described.
Fig. 1 is a perspective view of an exemplary fluid connector before use, with a membrane covering a connector opening, in accordance with some embodiments.
Fig. 2 is a longitudinal cross-sectional view of the fluid connector of Fig. 1, illustrating a connecting face of the coupling part provided in the connector.
Fig. 3A is a perspective view of the fluid connector of Fig. 1, with a different orientation.
Fig. 3B is a perspective view of the fluid connector of Fig. 1, with the membrane transparent and illustrating the front opening with the sealing interface and with two connecting members allowing a genderless use of the connector with another analog or identical connector.
Fig. 4A shows a connection system with two identical connectors mutually fastened, before removal of aseptic membranes and with valve position different in the connectors.
Fig. 4B shows the connection system of Fig. 4A, with illustration of latching means in a housing of a connector and an insert of the other connector protruding through this housing.
Fig. 4C also shows the connection system of Fig. 4A, with each actuation blocker set manually, by an associated sleeve, in a non-blocking position selectively obtained when the sleeve has been rotated to drive a valve plug in its plugging position, so that a disconnection of the system becomes available by a simultaneous pushing of the actuators once each valve assembly is in a closed state.
Fig. 5 is in exploded perspective view of the fluid connector of Fig. 1.
Fig. 6 is a view of the connection system of Fig. 4A, after removal of the membrane with a junction plane obtained at a contact area between two annular seals carried centrally by the connectors.
Fig. 7 is a longitudinal cross-sectional view of the connection system of Fig. 6, illustrating an open state for the valve assembly on the left, while the valve assembly on the right remains in closed state.
Fig. 8 is a perspective view of an exemplary embodiment of a tubular body of the connector with associated latching means associated with the female connecting member of the connector, here with a recess available to house the latching means in a peripheral region, so that a connector flange carries and retains the latching means.
Fig. 9 is a perspective view of an exemplary embodiment of a connector without any rotating actuator, with some pieces of the complementary connector illustrated to show position of these pieces in a connection system using two connectors of genderless type.
Fig. 10A is a perspective view of a locking slider according to an embodiment of the latching means.
Fig. 10B is a cross-section view of the inside of the flanged part of the connector, showing an abutment portion of the external actuator suitable to cooperate with an end part of the locking slider of the type shown in Fig. 10A.
Fig. 10C is a perspective rear view of a connector, similar to Fig. 4B but in a configuration of interest just before disconnecting a connection system, which is a configuration with the sleeve rotated to have plug lugs in the closest position relative to the flange, which means the plug is in a closing state and the external actuator is no more blocked by the actuation blocker that is now removed from the blocking position.
Fig. 11A is a perspective view of a connector according to another embodiment, having a male connecting member carrying a flexible part forming the insert fastening region.
Fig. 11BC is a longitudinal cross-sectional view of a connection system using coupling parts with a connector structure as in the connector of Fig. 11A, the latching means being included in a peripheral part/flange of the tubular body.
Fig. 11C is an exemplary piece to form the tubular body of the connector shown in Fig. 11A.

### MORE DETAILED DESCRIPTION

A detailed description of several embodiments of the invention is provided below, accompanied with examples and with reference to the drawings.
In the various figures, the same references are used to designate identical or similar elements.

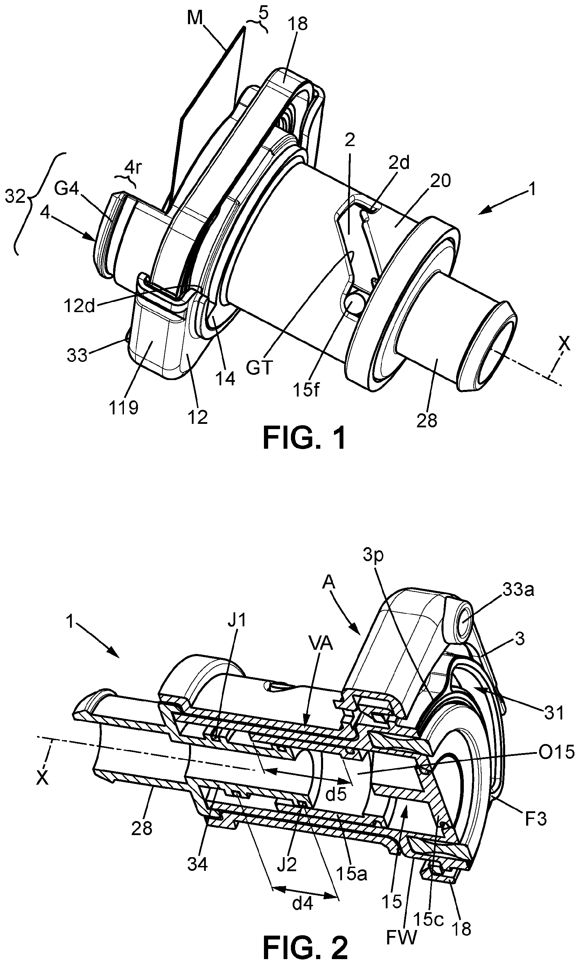

Referring to Figs 1-2 and 5, it is shown exemplary construction of a connector 1 that can be considered as sterilized, thus being treated by suitable sterilization technique, including gamma irradiation, E-beam, ethylene oxide (EtO) and/or autoclave technologies. The fluid connector 1 comprises a main body, which is a tubular body 2 suitable to delimit an interior space 7. The connector 1 is also provided with an aseptic attachment structure 5, including a removable membrane M. The tubular body 2 can delimit a fluid passage of constant section, possibly with an inner cylindrical surface for delimiting the interior space 7. A fluid passage is obtained by such interior space 7, with such fluid passage delimited by the tubular body 2 being rotationally symmetrical about a longitudinal axis X. Passage of the fluid conveyed through the fluidic connection using two interlocked connectors is allowed by having an interior space elongated that can house a plugging part of a valve assembly VA. The connector 1 also has a flange 3 that may extend peripherally around the cylindrical part of the tubular body 2. The removable membrane M prevents bio-contaminants and/or fine particles from passing through. The membrane M may be porous for gas.

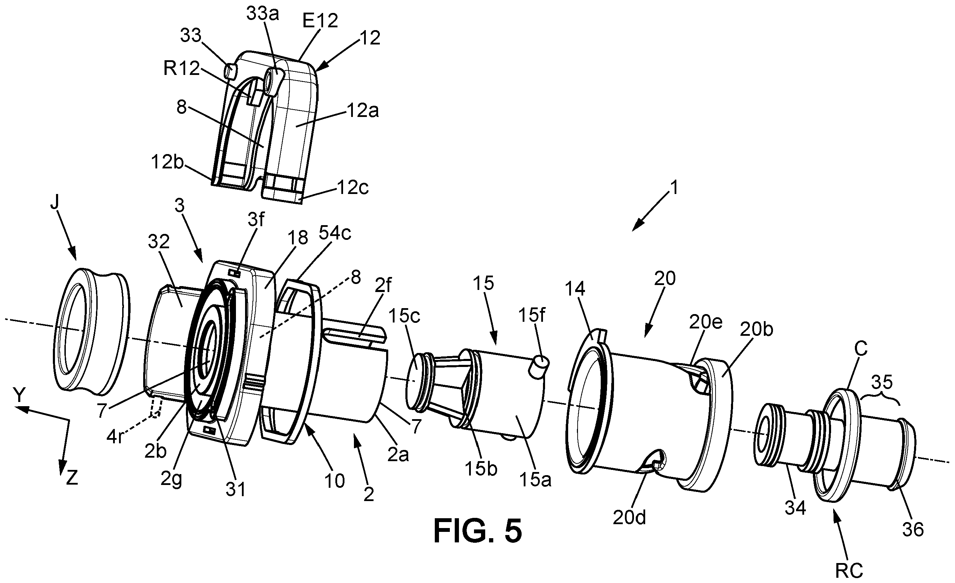

The flange 3 may be included in the tubular body 2 due to a one-piece construction (molded piece preferably), typically using plastic material such as any suitable rigid thermoplastic material. As shown in Fig. 1 the tubular body 2 may extend around a longitudinal axis X, while the flange 3 formed peripherally on the tubular body 2 also extends around the longitudinal axis X. The flange 3 is surrounding a front part of the cylindrical part and is provided with connecting members allowing a front connecting with another connector. More generally, the connector 1 has a longitudinal extension and the tubular body 2 may be provided with:
- a rear end 2a (at the opposite from the flange front face, as illustrated in Fig. 5); and
- a front end 2b with a central opening O.

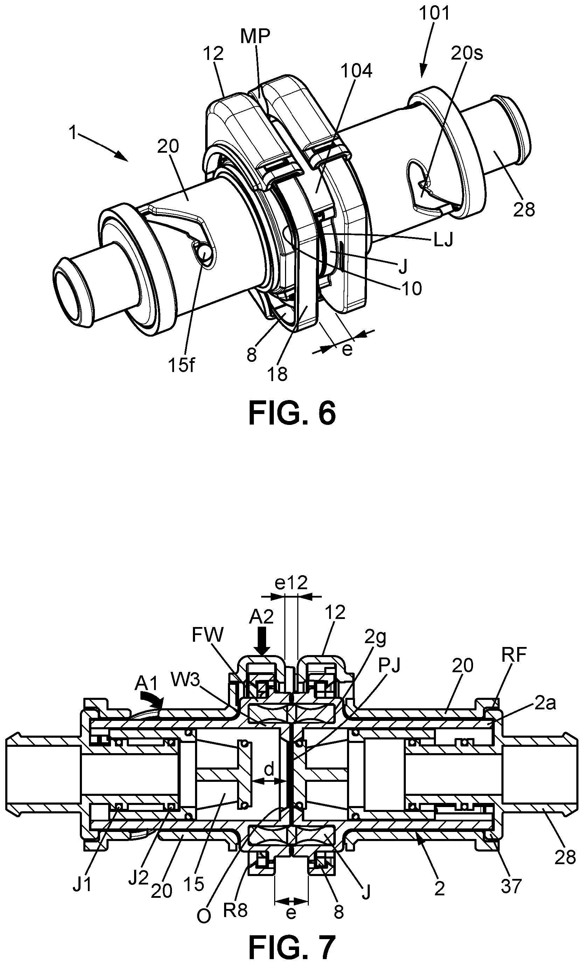

The flange 3, which can be molded from plastic material, can include an intermediate annular wall FW interposed (with a concentrical arrangement) between an inner flange side wall W3 and an external side wall 18, as shown for instance in Figs 2 and 7. Such configuration may provide a compact flange 3 with:
- a first groove of annular shape for a seal (at the front side), this front groove 2g being arranged between the intermediate annular wall FW and the inner flange side wall W3 that includes an end of the cylindrical inner face of the tubular body 2;
- and a second groove of annular shape (possibly rectangular), facing at an opposite side (rear side) as compared to the first groove, defining an annular recess where a locking of connection may be implemented.

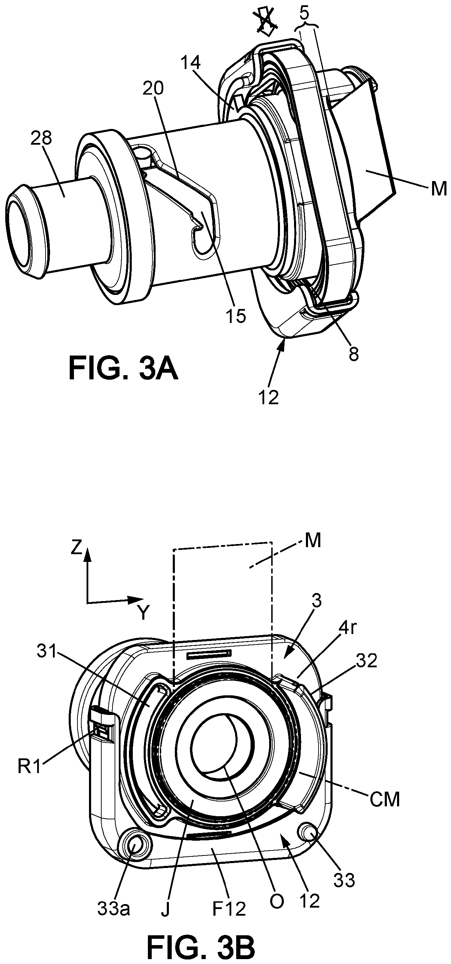

As shown in Figs 3A-3B, the flange 3 comprises a female connecting member 31 with an axial access 30 and a male connecting member 32 that is arranged on a different side of the flange, as apparent in the front view of Fig. 3B. The aseptic attachment structure 5 is formed by fastening the membrane M to an annular surface of the flange 3 at the flange front face, around the central opening O. Such attachment surface is covered by a margin portion CM that belongs to the covering portion of the membrane M. The margin portion CM may typically extend around an annular seal J received in a groove 2g (front groove) of the flange 3. A complementary portion of the membrane M, possibly of rectangular shape, may extend radially outward from the covering portion to a pull end part that is accessible for the operator.

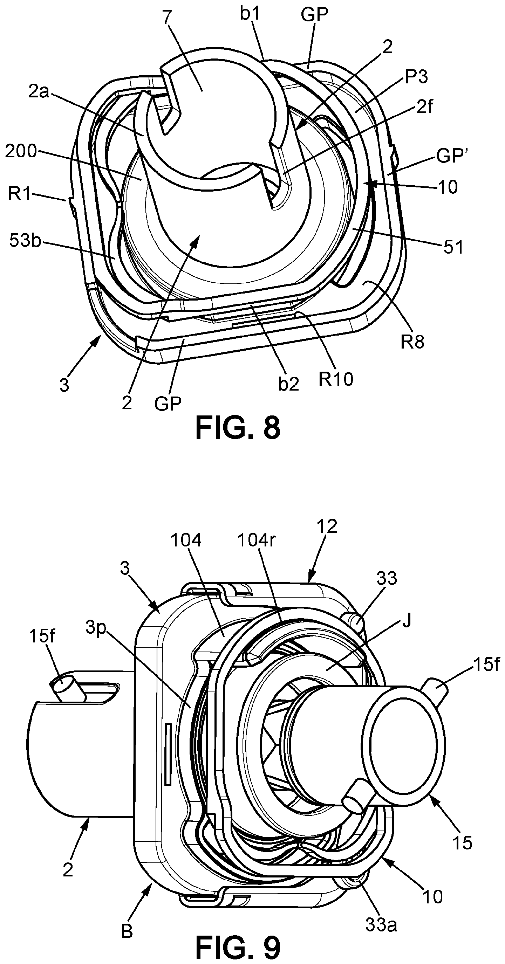

As apparent in Figs 1-2 and 3A-3B, the flange front face can be partly covered by the membrane M, with the female connecting member 31 the male connecting member 32 distributed on either side relative to the membrane M. The male connecting member 32 protrudes from the flange front face and may follow a curved section of the attachment surface and/or be adjacent to the margin portion CM. As illustrated in Figs 1 and 2, the flange 3 may have a thickness that is superior to a length of the seals J surrounding the opening O. At the front of the flange 3, a projection 3p of annular shape (Fig. 9) may be provided, allowing the seals J to be in contact for a tight connection at a front contact area matching with this projection 3p on the one hand, while also having a spacing or axial gap e maintained between two peripheral portions of the flanges 3. The (female and male) connecting members 31, 32 may be disposed close to the central opening O, possibly locally increasing the radial extension of such projection 3p. Such arrangement can facilitate a manual actuation of the disconnection, with a pushing or any suitable action to be exerted from one side of the flange 3, typically using an external actuator 12 provided with an end actuation portion E12 and side portions or arms 12a, one of which can be interposed between the flanges 3 to partly cover a flange peripheral portion.

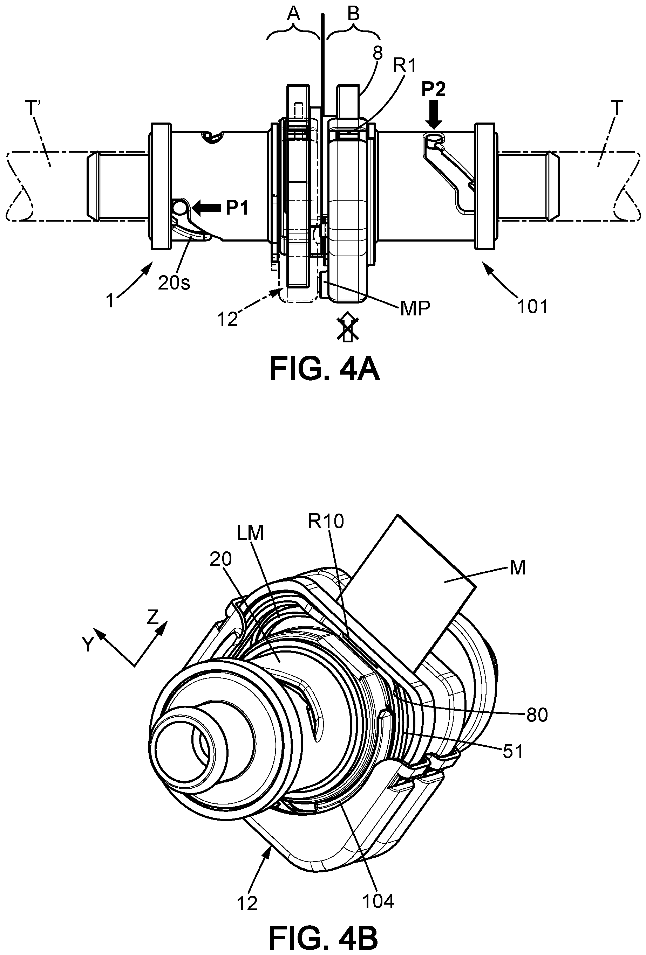

In some embodiments, a curved profile is used for an inserting tab 4 of the male connecting member 32 and a curved shape can be used as well for the axial access 30 delimited by the female connecting member 31. The size and shape of each profile, for the inserting tab 4 and the axial access 30 may be matching so that the connector 1 can cooperate with an identical connector 101 having same connecting members, female connecting members 32. FIG. 4A and 4B show a connection system with the two connectors 1, 101 coupled at coupling parts A, B that are of identical structure. Each coupling part A or B may include a flange 3 as above disclosed, with the aseptic attachment structure 5.

In a connection system as shown for instance in Figs 4A-4B, each membrane M can be removed with the other one, radially by a pulling action. Each membrane pull end part may extend beyond the flanges 3. Pulling of the two membranes M, simultaneously, can be performed along a first transverse direction Z that is perpendicular to the longitudinal axis X of the tubular body 2. Insertion of the inserts or inserting tabs 4, 104 formed by the male connecting member 32 may be a full insertion, into the axial access 30 in the facing flange, to allow an axial contact between the front open ends 2b of the tubular bodies 2, without axial interspace at a central region of the connection system. The annular seals J thus can be in axial contact after removal of the membranes M.

Figs 6 and 7 show that a management of fluid circulation in the fluid connection system combining the two connectors 1, 101 can be obtained, using a valve assembly VA in each of the connectors 1, 101. When reaching full insertion of the inserts of the male connecting members, a locking can be obtained using latching means LM that can be arranged at the rear of each flange 3. Then, after such mechanical connection, the membranes M can be removed to obtain a fluid communication. This may be allowed by having an initial position of the plugs 15 of the valve assembly VA in retracted state, i.e. in an opening position with each plug 15 arranged away from the junction plane PJ obtained at interface between the seals J.

### Valve assembly in connector

Now, referring to Figs 1-5, a valve assembly VA is provided with a plug 15 adapted to be displaced in the interior space 7, between a retracted rear position, distal from the central opening O at the front end 2b, and a closing position where the plug 15 is in annular sealing contact with an annular rim or any suitable seating region provided around the opening O. The seating region may be provided at the front end 2b in an annular surface facing rearwardly. As shown in Fig. 5, the plug 15 may extend between a rear axial end 15a, preferably of annular shape to delimit a flow passage, and a front closing portion 15c. The plug 15 is also provided with an intermediate portion making a transition between the closing portion 15c that can be a radial portion and an annular section (for instance cylindrical) provided in the axial end 15a that extends annular around the longitudinal axis X. The plug 15 may be a single piece, except one or more possible sealing member(s) defining an outer circumference at the closing portion 15c.

The intermediate portion may be a cross portion 15b that interconnects the tubular rear portion of the plug 15, which is a sliding portion adapted to slide along an inner face of the tubular body 2. Referring to Figs 5, 7 and 9, the plug 15 may define a flow passage delimited by the tubular rear portion. This passage axially opens in a partitioned chamber of the interior space 7, which is partitioned (possibly divided in four fluid passage sections) by the cross portion 15b arranged as a partitioning wall, here having a X-profile. With such arrangement, in open state of the valve assembly VA, flow can pass though the flow passage and then pass around the closing portion 15 that may be located in a chamber of wide section (wider than a diameter of the central opening O), as apparent for instance in Fig. 7, to finally reach the opened junction at the junction plane PJ.

With such plug 15 movable to reach the junction plane PJ, dead leg or any significant interspace is prevented. Actuation of the plug 15 can be a manual actuation, using a movable part distinct from the flange 3 and arranged at the rear of the flange 3. This movable part can be a sleeve 20.
Referring to Fig. 5, the sleeve 20 may be supported on a cylindrical rear portion of the tubular body 2. The sleeve 20 can be mounted at the rear side on the tubular body 2, possibly some inner recesses or slots to have a sleeve front section adapted to pass over the rear axial end 15 of the plug 15, which may be an end provided with lugs or at least suitable protruding part that can be a cam follower when having a bayonet connection for driving the plug 15.

The valve assembly VA may thus be assembled using three components: the plug 15, the cylindrical part of the tubular body 2 and the sleeve 20 that surrounds the tubular body 2 (thus also surrounding the plug). This is a simple arrangement, with any spring member. Each of the sleeve 20 and the plug 15 may be in contact with a same piece forming the tubular body 2, with this piece simultaneously guiding the plug 15 and any actuation movement(s) of the sleeve 20.

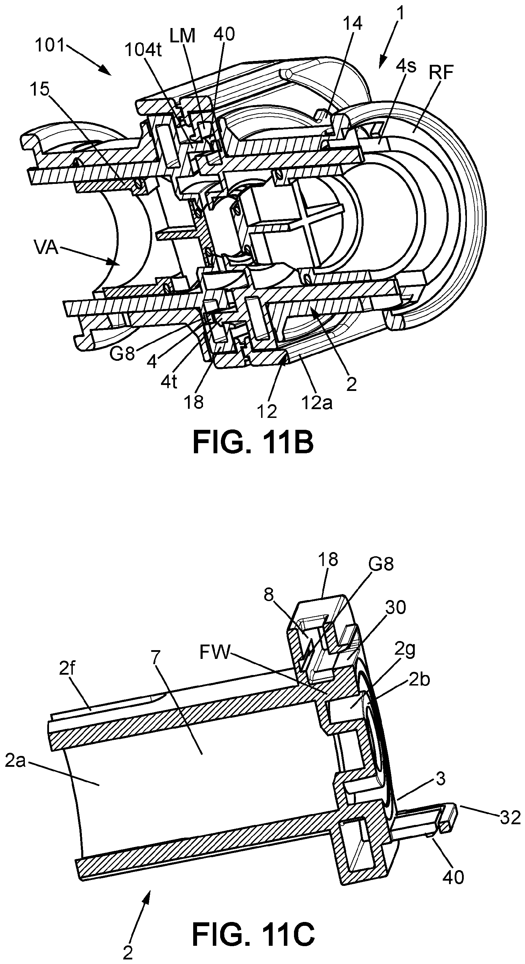

In some options, the connector 1 can axially maintain the sleeve 20 between two shoulders, one of which is a shoulder part 200 provided on the rear flange face, corresponding to a bottom of the groove 2g for housing the seal J, the other shoulder being a collar C of an end fitting RC. In other options, a shoulder can be directly formed by planar rear face of the flange 3, as apparent in Figs 11B-11C. A barbed end 28 or similar nozzle may optionally be provided as an axial extension of the tubular body rear part, at the rear of the connector 1. In some variants, such barbed end 28 may be included in a tubular piece that connects a flange piece forming the peripheral flange 3.
Referring to Figs 5, 6, and 7, it can be seen that the end fitting RC may have an inserted portion 34, inserted through the sleeve 20 and in inside the rear axial end 15a of the plug 15. The inserted portion 34 remains stationary relative to the sleeve 20, by being secured fixedly at the rear end 2a of the tubular body 2, at a rear annular fixation region RF. An axially protruding rim of the collar C may form the shoulder preventing any rear movement of the sleeve 20. In such kind of assembling, the sleeve is a rotation actuator able to drive the plug 15 along direction of the longitudinal axis X.

The inserted portion 34 can bear two annular seals J1, J2 and is suitable to guide the sliding portion/rear end 15a of the plug 15. While only the front seal J2 distal to the collar C is in tight contact with the plug 15 is the closing position, as illustrated on the right in Fig. 7, the two seals J1, J2 (with the seal J1 being proximal to the collar C and axially spaced from the seal J2) can be in contact with inner cylindrical face of the sliding portion of the plug 15, when the plug 15 is retracted to release the opening O. The stroke of the plug 15 may correspond to an axial distance d lower than distance d4 between the two seals J1, J2.
Referring to Fig. 2, it can also be seen that the sliding portion of the plug 15, including the rear end 15a, may have a length d5 greater than the distance d4, so that tightness is optimized. Indeed, in open state of the valve assembly VA (as shown on the left in Fig. 7), there are two distinct sealing areas, using the seals J1 and J2 contacting the plug sliding portion, when biopharmaceutical fluid is flowing through such connector 1 each connector (1 or 101) has an insert or inserting tab (104 or 4, respectively) protruding inside a rear recess of a connector housing 8. Fig. 4B-4C illustrate an exemplary inserting tab 104 provided in the coupling part B of a connector 101, which is inserted to protrude at the rear side of the flange 3 of the coupling part A provided in the connector 1. A fastening region 104r of the insert 104 and a fastening region 4r of the insert 4 can be engaged to lock the fluid connection as described later. involved in a fluid connection system. A seal may also be provided around a plug annular part surrounding the cross section 15b, for instance with this seal received in a circumferential groove, to have tight radial contact between the inner face of the tubular body rear part and the plug 15.

The valve assembly VA is easy to be controlled. To prevent accidental driving of the valve assembly VA, a retaining effect may be implemented in the connector 1 for better maintaining a given position of the plug 15. As apparent in Figs 1, 3A and 4A, position P1 of the release of the opening O and/or position P2 for the closing position may be associated to a relief, tooth or similar member having a retaining effect, so that starting movement of the sleeve 20 from at least one of these positions P1, P2 requires an additional effort.

In the following, a bayonet connection with a rotation action to drive the plug 15 is disclosed. However, it is understood a screwing, or any other kind of actuation, can also be satisfactory, in order to drive position of the plug 15 that extends in the interior space 7 while also having protrusion(s) or peripheral part(s) involved in a mechanical driving from the outside of the valve assembly VA. Besides, it is observed that the plug 15, the valve assembly VA or the connector 1 taken as a whole, in preferred embodiments, may be provided without any metal part.

In some embodiments, the connection sequence for mating the connector 1 to a second compatible aseptic connector 101 is as follows. First, each connector 1, 101 can be provided in assembled state, with the valve assembly VA open, which means the plug 15 is retracted and away from the membrane M of the aseptic attachment structure 5. When having lug(s) 15f or protruding part(s) for controlling movement of the plug 15, such protruding part(s) may be positioned by default away from a front end of the guiding track GT (with the opening O initially only covered externally by the membrane M), while the plug 15 is retracted away from the front opening area. The plug 15 can be in such retracted state, i.e. kept away from the sealing position, in all upstream phases (manufacturing/transporting/handling) of the connection system involving two connectors 1, 101. Possibly, at least a first tooth or similar retainer is used at rear end 20e of the guiding track GT to prevent accidental sliding of the plug 15 in such above-mentioned phases.

Each connector 1, 101 may be genderless, with just a difference in orientation to have each male connecting member 32 facing a female connecting member 31, with the two flanges 3 moved closer until locking the connection by latching means LM that can be at a periphery of or surround the tubular body rear part.

Then, after mating the coupling parts A, B, using the aseptic attachment structure 5, an aseptic connection is made between the two connectors 1, 101. After removal of the membranes M, with the seals J in mutual contact, there is no requirement for the user to actuate the sleeve 20 since each valve assembly VA is already open. In some variants, one of the connectors used in the fluid connection may be arranged with a plug assembled differently, for instance with a plug that is spring biased toward the positions P1, P2 and ability to be manually driven against the return effect of the spring. A bayonet connection in a given connector 1 can thus be compatible with a connector having a different way of driving the plug 15, provided the coupling parts A and B are compatible.

### Bayonet connection

Referring to Figs 1, 5-6 and 11A, the connector 1 has a single piece acting a manually operable member for driving the plug 15. This piece may have a graspable annular section and forms a sleeve 20. In some embodiments, the sleeve 20 can be rotated to open and/or close the plug as many times as the operator/user desires. In other embodiments, for instance with a tooth or similar retaining member 2d included in the sleeve 20, actuation of the closure by reaching the second position P2 (see Fig. 4A) is the sole rotation allowed. In such a case, the plug 15 can be retained and/or locked via a lug 15f carried by the plug 15. The tooth or detent element 2d shown in Fig. 4A for position P2 illustrates a definitively locked position, which is normally obtained only when having a connection system already used, with no more need for a flow through the connector 1, 101 and associated flexible hose T' or T (or through a suitable fluid circuit component connected at the rear of the connector 1, 101).

One or more grooves or slots 20s, 20s may be provided on the sleeve 20. Referring to Figs 1 and 5, each groove or slot 20s may extend on an angular sector that is more than 40° and less than 110°, preferably of about 90°. More generally, any suitable guiding track GT may be formed on the sleeve 20, to form a bayonet connection, with a lug 15f or similar protrusion of the plug 15 engaged with or through the guiding track GT. This allows the connector 1 to be firmly taken in hand at the flange area (possibly with a grasping at the external actuator 12 that is mounted on the flange 3), while selectively rotating the sleeve 20 around the tubular body 2. Possibly, an operator may also exert a twisting, with the tubular body 2 (grasped at the flange area for instance) and the sleeve 20 being rotated in opposite directions.

Each protruding part or lug 15f may act as a slidable detent element, slidable along a slot 2f provided in the tubular body 2, as well apparent in Figs 5 and 8. Any suitable anti-rotation part may be used to have the lug(s) 15 not rotating around the longitudinal axis X. Optionally, the slot 2f (distinct from the bayonet slot(s) 20s possibly forming the guiding track GT) may be a rectilinear slot that is adjacent to the first open end 2a of the tubular body 2, provided that the sleeve 20 can rotate (typically without any longitudinal displacement), here around the tubular body 2.
Referring to Fig. 5, the track rear ends 20e, provided at the rear of the slots 20s or similar guiding parts of the guiding track GT, may be arranged with a provisional retaining effect, for instance with a local width reduction of the slots 20s, so that each lug 15f can pass with a friction over the local restriction at the track rear ends 20e. A first tooth may be formed for having a small restriction in the lug passage delimited in the sleeve 20.

In options with a tooth or detent element 2d at the opposite end (front end 20d) of the slots 20s, it is understood that the tooth/detent element 2d may be of sufficient size to prevent any return of the plug 15 toward an open state. The retaining/detent element 2d may be a tooth or similar protrusion, angled relative to an edge of the guiding track end, for instance forming an angle of about 90° only at the tooth side adjacent to this guiding track end.
Such relief/tooth may form a second tooth when a first tooth is present at the opposite in the slot 20s. In embodiments, with at least one tooth or detent element 2d at the front end 20d of the bayonet slot 20s (second tooth to prevent any connector of the system/assembly (assembly of two identical connectors, preferably, forming the fluid connection) from being reused in an uncontrolled and therefore non-aseptic way (connections/disconnections can still be made with the connecting members 31, 32, but the fluid passage cannot be re-opened). Referring to Figs 1, 3A, 4A and 6, two notching teeth or similar detent elements 2d on each bayonet slot 20s can be included, to enhance the retaining effect at the position P2 and thus prohibit a non-aseptic re-opening. Position P1 can be a relatively stabilized position, using a comparatively smaller tooth (first tooth), helping in keeping the piston/plug 15 retracted. In some options, markings may be provided to indicate the positions P1, P2 and/or indicate a direction to rotate the sleeve 20 in order to move the plug 15 toward the closing position. Optionally, a marking may be provided for rotation direction allowing reverse movement, i.e. toward the open state of the valve assembly VA. In the illustrated embodiments, the rear/retracted position of the plug 15 is a position axially away from the front opening O.

When using a bayonet connection, the plug 15 may be provided with any suitable cam follower, possibly forming the lug 15f or other protruding part formed on a side wall of the plug 15. Use of a collar or rear shoulder 20b at the rear of the sleeve 20, possibly with additional reliefs or gripping surface portions, may facilitate the gripping of the sleeve in some options. The bayonet actuation can be combined with rotation of an actuation blocker 14 (visible on Fig. 5 or Fig. 11B) that interacts with an external actuator 12 that can control the latching means LM or control a flexible part (flexible member 4t in Fig. 11C) in the insert of the connecting male member 32. Possibly, the closing portion 15c of the plug 15 may remain closer from the tubular body front end than the sleeve front end, even in the retracted position.

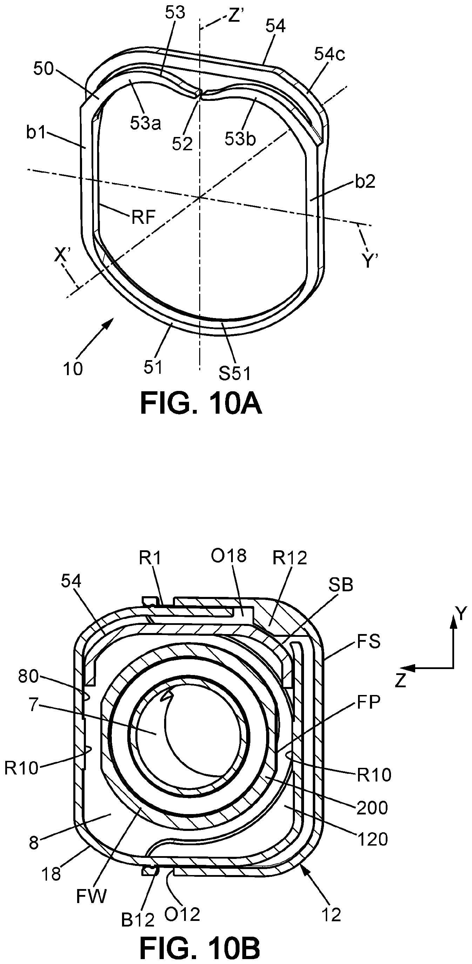

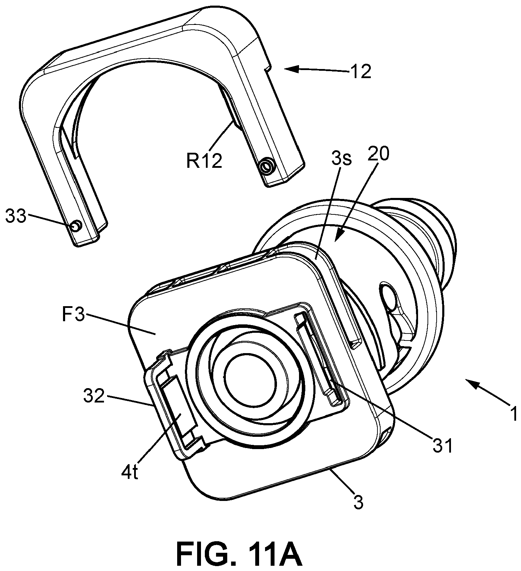

The engagement with latching means LM, providing a retaining effect at the fastening region 4r or 104r, cannot be released accidentally. To allow disconnection, simultaneous operation should be performed to remove the retaining contacts. Each retaining contact is suppressed by having one/at least one amongst the latching means LM and the flexible engagement insert part of the connecting male member 32 displaced (typically along a transverse direction) by a suitable part or abutment member R12 such as in the example of Fig. 10B or Fig. 11A, which is a part R12 carried or integral with the external actuator 12. Blocking the external actuator 12 by an actuation blocker 14 driven at same time as the plug 15 can offer a simple gesture to obtain the closing of the opening O and removal of the blocking state (for the external actuator 12). Since two connectors are interlocked, the release implies disengagement with all of the latching means LM, thus preventing any involuntary disconnection.

A full removal of the actuation blocker 14 may be obtained when fully rotating the sleeve 20 to reach the position P2, i.e. possibly an irreversible closing position.
The external actuator 12 may be clipped, using lugs or outer reliefs R1 that locally protruding from the external side wall 18. For instance, the reliefs R1 are arranged at opposite side along the Y-direction and configured to allow, after mounting, a further displacement of the external actuator 12, so that a sliding is obtained in response to pushing actuation on a proper pushing surface PS at an accessible end E12 of the external actuator 12.

### Position-locking system

Referring to Figs 5, 7-9, 10A-10B and 11B-11C, it can be seen a housing 8 is defined on the flange 3 (and around the plug 15), radially beyond the groove 2g for the seal J, so that latching means LM may be carried (preferably inside the housing 8) by the flange 3 that also carries an external actuator 12. While the external actuator 12 has here a structure of a push button with two arms 12aforming two parallel sliding sides for having a mobility on the flange 3, other constructions may be used, possibly using a hinged actuator or any suitable structure with a movable part.
The external actuator 12 may be C-shaped, U-shaped and/or configured to cover three distinct sides of the flange 3, while being slidably mounted relative to the flange 3. When the external actuator 12 comprises two sliding arms 12a, the female connecting member 31 and the male connecting member 32 can extend each in an intermediate position between:
- the intermediate wall FW that surrounds the seal J,
- and one of the sliding arms 12a.

Referring to Figs 6-7 and 9, in the connection system, each male connecting member 32 is clipped in the housing 8 provided beyond the transverse wall of the flange 3 crossed by the inserting tab 4, 104 of the male connecting member 32. The width of each inserting tab 4, 104 may be superior or equal to a width of the flange 3 and at least one bevelling may be provided at a free end of the inserting tab 4, 104, to help in having a guided insertion, simultaneously, of the inserting tabs 4, 104, in opposite directions. The locking is also performed simultaneously and automatically, using for instance same locking slider 10 interfering at an area of the annular recess R8. At this area, the axial access 30 opens for the passage of a fastening region 4r, 104r orientated radially outward on each inserting tab end. The retaining/interfering portion 51 of the locking slider 10 may also have a ramp or beveled surface S51, intended to facilitate the insertion movement of the male connecting member 32 which must move the locking slider 10 radially outwards until the retaining annular rim (typically rim of a groove G4) has passed beyond the interfering portion 51, which then causes the locking slider 10 to rise in the groove/to move centripetally due to the return forces associated to or integrated to the locking slider 10, possibly using flexible tab(s) 53a, 53b (see Figs 4B and 10A).

For having such locking action, the locking slider 10 is shown as an exemplary piece forming the latching means LM, using an annular shape fitting with the annular shape of the annular recess R8, possibly using retaining tabs R10 for only allowing a sliding of the locking slider 10. Of course, other structures may form the latching means LM, for instance using a recess arranged only in a given angular sector in the flange 3, where the inserting tab or insert to be engaged extends. This angular sector may correspond to the side where a pushing is exerted on the external actuator 12.

In the connection system, the two connectors 1, 101 are coupled so that the external actuators 12 are both pushable from a same side, along same transverse direction Z. In some options, there is an interconnection linking the external connector 12 of the first connector 1 to the external connector 12 of the second connector 101, using synchronization means MP for keeping same position of the external actuators 12 relative to the longitudinal axis X. Referring to Figs 4A and 5, the synchronization means MP may include a structure with two pins 33 and two pin receivers 33a or any similar structure distributed at two locations on the mutually facing front walls F12, which are actuator faces separated from the distance e12 as shown in Fig. 7. Having two axial links between the external actuators, extending in the corresponding interspace, can preventing any relative rotation between the external actuators 12.

In embodiments, due to synchronization effect, a pushing as reflected by arrow A2 can practically exerted for obtaining disconnection, after properly rotating the sleeve 20 along direction A1 (shown on the left in Fig. 7) to have the two plugs 15 in closing positions. Here a left rotation (direction A1) on the sleeve 20, for a bayonet configuration as reflected by the lug position in Fig. 3A, is required to allow release of the corresponding actuator 12 that was blocked so far, by the actuation blocker 14. The arrow in Fig. 3A reflects the pushing is not available in this state of the bayonet connection, which means the two sleeves 20 provided in a connection system with two connectors 1, 101 must be set in the proper release configuration with the closing position of the two plugs for allowing disconnection.

Now referring to Figs 5, 8, 9 and 10A-10B, an exemplary locking slider 10 will be described.
Stability of the connection system is obtained by latching means LM that are each stably maintained in the housing 8, in order to avoid accidental disconnection due to accidental removal of the male connecting members 32. With an annular shape using four narrow sides (of low thickness), the latching means may be formed with optimized use of plastic material. A locking slider 10 is of interest to ensure a combined retaining action and sliding action, around the projection/shoulder part 200. The retaining tabs R10 may form means for definitively retaining the locking slider 10.

In some options, a slot 3f (see Fig. 5) or recess separates each of the retaining tabs R10 from a radial portion of the flange 3, which may provide locally a hinge effect (helping in having tab(s) R10 slightly deviated radially outward when inserting the locking slider 10 to have the locking slider 10 extending in a transverse configuration along the bottom wall P3, preferably perpendicular to the longitudinal axis X. Fig. 8 illustrates the locking slider 10 just before a clipping, using a pair of retaining tabs R10: one protruding radially inward to block the arm b1, the other one protruding radially inward in opposite direction (transverse protruding direction of the tabs R10, parallel to Z-direction) to block the arm b2, whereby the arms b1, b2 can slide along the Y-direction. The locking slider 10 cannot move along direction of the longitudinal axis X, and cannot move along Z-direction due to sizing/external width of the locking slider 10 along its left-right transverse direction Y', which is parallel to Z-direction in mounted state.

Referring to Fig. 10A, it can be seen that the locking slider 10, or any similar annular suitable piece of the latching means LM, may be an unlockable locking element, provided with an outer part, possibly annular with a symmetry axis Z', so that the two straight sliding sides b1, b2 are at equal distance from such axis Z'. This axis Z' can match with Y-direction of the connector 1 in mounted state of the locking slider 10.
The locking slider 10 may have a slot 52 at an end that corresponds to a pushing side of the external actuator 12, the slot 52 separating two flexible tabs 53a, 53b. More generally, a deformable portion can be provided, preferably axially offset relative to a contact portion, called bridge portion 54, driven by the external actuator 12. The flexible

Referring to Figs 10A-10B, the contact/bridge portion 54 may also extend, transversely, at the same end of the locking slider 10 as the flexible tabs 53a, 53b but with an axial offset along Z'-direction. The bridge portion 54 may extend radially farther from the longitudinal axis X and may also be offset along a central axis X' of the locking slider (parallel to X-direction). The bridge portion 54 thus may be rearwardly offset as compared to the elastic return part 53, here provided with the tabs 53a, 53b. A junction 50 can be provided between the bridge portion 54 and the arms b1, b2, to have the locking slider 10 formed as a closed loop. The flexible tabs 53a, 53b may extend each from an arm end to a free end delimiting a side of the slot 52.

In advantageous options, the two flexible tabs 53a, 53b can be arranged symmetrically with respect to the X'Z' plane. In addition, the flexible tabs 53a, 53b are arranged (along Z'-direction) under the bridge portion 54. Typically, in mounted state, they are interposed between the bridge portion 54 and a continuously curved surface of the intermediate wall FW. In addition, one will note that each of the flexible tabs 53a, 53b has a curvature oriented away from a central region of the bridge portion 54 (curving downwards in non-limiting embodiment of Fig. 10A). In addition, each of the flexible tabs 53a, 53b optionally has a cross-section or thickness which decreases from its root to its free end (where the slot 52 is delimited).

Referring to Figs 8 and 10A-10B, the elastic return part 53 may be configured to be in radial contact against the shoulder part 200. For the connection, when an inserting tab 4 or 104 engages on the interfering portion 51 provided in the annular recess R8, starting to axially slide on beveled surface s51 provided at beveled front side of the interfering portion 51, a clipping is obtained once the interfering portion 51 reaches the groove G4 as illustrated in Fig. 4B. It is understood that, once the membrane(s) M is no more present due to a connection, the locking action by the latching means LM, for instance using the locking slider 10 in each connector 1, 101 of the connection system, is obtained by exerting an axial pressure on each O-ring or seal J to ensure a sealing function around the central passage defined at the openings O. The sealing elements or seals J may have an excess in length to ensure an axial pressure is exerted in such annular contact area, forming an annular junction (Fig. 6).

The insertion movement is completed when having the locking slider 10 engaged in this groove G4 that forms all or part of the fastening region 4r, 104r. Figs 6-8 and 9 show that the inserting tab 104 (idem for inserting tab 4), passing through the axial access 30, can thus be interposed radially between the intermediate wall FW (forming the circumferential outer surface of the shoulder part 200) and the interfering portion 51 of the locking slider 10. The elasticreturn part 53 prevents radial displacement of the interfering portion 51, unless the locking slider 10 is displaced along the slide 80 by a pushing action exerted at the pushing surface PS.

The two flexible tabs 53a, 53b may be formed integrally with the locking slider 10, the flexible tabs returning the locking slider 10 toward a first position, referred to as the locking position, with the interfering position being proximal to the longitudinal axis (FIGS. 4B-4C), the fastening region 4r or 104r being retained by the retaining an interfering portion 51, here curved with same profile as the groove G4 provided in the fastening region 4r, 104r. The two flexible tabs 53a, 53b are adapted to flex to allow the plate or similar piece of the locking slider 10 to move to the second position, referred to as the unlocking position, which corresponds to move the bridge portion 54 closer to the longitudinal axis and shift the interfering portion 51 to an external/distal position with no more engagement in the groove G4, so that extracting the connectors, simultaneously, is allowed.

More generally, in the connector 1, the latching means LM are provided for locking the inserted position of the male connecting member 32 of a compatible complementary connector 101. Fig. 4B shows a slide 80 can be formed at the interspace between the external side wall 18 and the projection 200. Since a housing 8 is provided in the tubular body 2, typically using an annular interspace or recess R8 that is suitably sized and shaped to form two guiding sides GP of the slide 80, the assembly of the connector 1 may be simple, possibly without requiring any screwing member or screwing action. The two guiding sides GP, here at an inner face of the external side wall 18, may be parallel to the transverse direction Y as shown in Fig. 3B. When having a locking slider 10 as shown in Figs 10A-10B, the arms b1, b2, may cooperate with the slide 80. Flat or planar short sides FP may be provided in the shoulder part 200, locally thinning the intermediate wall FW, as shown in Fig .10B, so that the sliding arms b1, b2 may be easily movable along a transvers plane parallel to plane YZ.

The flange 3 may carry the seal J at the flange front face, while also having the housing 8 or peripheral region delimited, at the flange rear side, by the external side wall 18. When forming an annular recess R8, the intermediate annular wall FW may be arranged as a rear annular protrusion delimiting the recess R8 with the external side wall 18. The locking slider 10 or any suitable latching means LM may be a latch formed in a single piece of annular shape, sufficiently flat to be entirely housed in the annular recess R8, i.e. without protruding outside the flange 3 at the rear side, as apparent in Fig. 4B.

Now referring to Figs 3A-3B, 4A-4C, 8, 9 and 10A-10B, it can be seen the external side wall 18, possibly rectangular, may delimit the recess R8 so that this recess R8 is designed as an annular (possibly rectangular) groove around an annular projection 200 or similar base of the tubular body 2 that protrudes from a bottom wall P3 of the flange 3, in same direction as the external side wall 18. The locking slider 10 may be hidden inside such recess R8.

The bottom wall P3, the external side wall 18 and the intermediate wall FW can form the housing 8, made integral with the tubular body 2. Referring to Fig. 8, the external wall 18 can provide two parallel guide portions GP and two straight side portions are included in the projection 200, forming opposite parallel sides, all extending along a same transverse direction Y.
Referring to Fig. 10A, symmetry axis Z' of the locking slider 10, may optionally define a length direction of the locking slider 10, while a particular corner 54c (being exception in a full symmetry of the locking slider 10) formed at an end of the bridge portion 54, on a given side along Z-direction which is the pushing surface side, defines an abutment surface SB in contact with an offset abutment member R12 provided as an inward protrusion in the external actuator 12.

More generally, the bridge portion 54, possibly offset at the rear of an annular portion of substantially constant thickness, may be the driving part of the locking slider 10, which is pushed at a contact region, forming the abutment surface SB that is facing outwardly. In a given connector 1, the bridge portion 54 may be, at the side of the male connecting member 32, the portion that is actuated to simultaneously drive the two sliding arms b1, b2 in a sliding movement, knowing these two sliding arms b1, b2 form parallel sides of the locking slider 10. While embodiments show a locking slider 10 of annular shape, other structures may be provided, for instance using a U-shape of the locking slider with arm end providing the engagement parts to interact with a fastening region 4r, 104r of an insert passing through the axial access 30.

Whatever the way the bridge portion 54, in contact with an abutment member R12 of the external actuator 12, is arranged at a first end of the locking slider 10, the two slider sides b1, b2 may join the bridge portion 54 at corner regions with the bridge portion 54 being for instance elongated to extend along a side of the external side wall 18 that is open at a radial opening 018. Fig. 10B shows an exemplary circumference of the external side wall 18, with the opening 018 allowing the abutment member R12 to engage the locking slider 10.

The driving surface for contact with latching means LM and the latching means LM may be essentially or totally integrated in a concealed area, inwardly with respect to the external side wall 18 and the external actuator 12 for instance. Arrangement of the external actuator 12, outside the flange 3, allow this actuator to be easily accessible, while being operatively coupled to hidden latching means LM. Displacement of the external actuator 12, for instance when pushed along Z-direction when allowed due to the sealing position of the plugs 15, causes release of the engagement between the latching means LM and received insert/inserting tab.

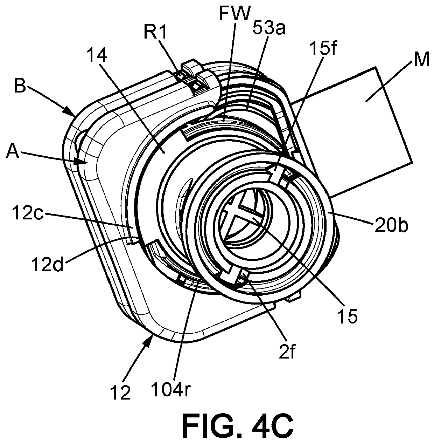

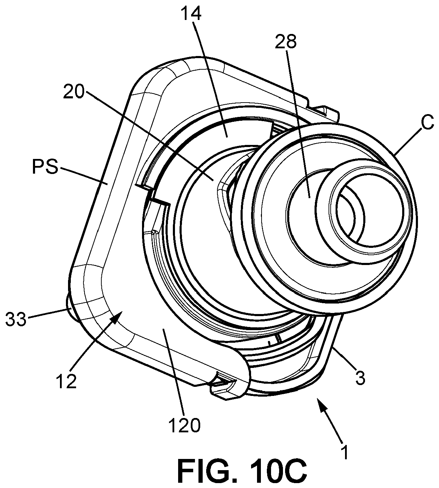

Regarding the actuation blocker 14, this part may be a partial collar or similar relief of the sleeve 20, radially facing an abutment part 12d, possibly formed as an axial protrusion, of the external actuator. When having the sleeve 20 rotatable, the actuation blocker 14 occupies an angular sector that can be substantially equal or inferior to angular stroke of the sleeve 20 when passing from the retracted position of the plug 15 (which may be an initial position, for instance when the membrane is still present) to the sealing/closing position. The actuation blocker 14, radially adjacent to the abutment part 12d in retracted state of the plug 15, prevents the displacement of the external actuator 12, as shown for instance in Fig 4C or 10C. Since the sleeve 20 is rotatably movable to occupy the second position as shown in Fig. 1, in which the actuation blocker 14 is offset relative to the abutment part 12d, there is a second actuation blocker position releasing the external actuator 12.

Using the pins 33 and pin receivers 33a or similar linking part, unlocking and disconnection of the fluid connection is allowed when displacement of each of external actuator 12 is allowed, which means each sleeve 20 has reached a second position such as shown in Fig. 1.
Use of two slides 80, in each connector 1, 101 of the connection system such as shown in Figs 4A and 7, allow forming relatively flat flanges 3 since each slide extending in a transverse plane YZ perpendicular to the longitudinal axis X. Moreover, mounting of the locking slider 10 by the rear face of the flange is of interest to have an efficient connection with appropriate sealing contacts and proper aseptic attachment structure 5.The locking slider 10, possibly formed as a molded plastic part comprising a wide open locking plate, may be a flat piece (compact piece along longitudinal axis X), with a central opening through which a part of the intermediate wall FW of the flange can pass.

In the depicted embodiments, the external actuator 12 is distinct from the locking slider 10. In some variants, a transverse extension of the locking slider can be used for providing a pushing surface or forming an actuator, possibly using a slot in the external side wall for having a pushbutton or similar actuator integrally formed with the plate of the locking slider 10.

The tubular body 2, the plug 15 and/or the external actuator 12 in the connectors 1 and 101 can be obtained by molding a plastic material. In particular, the tubular body 2 and the plug 15 in particular can be formed of plastic materials biocompatible with biopharmaceutical substances, for example polypropylene, polyethylene, polycarbonate, polysulfone, polyvinylidene chloride, polyethylenimine.

### Option(s) without any intermediate piece between the external actuator and the insert

While a locking slider 10 may be used in many embodiments, the connector 1 can alternatively allow a locking action when the mechanical connection between the connectors 1, 101 is completed, only by interaction between the flanges 3 provided in these connectors 1, 101. In such case, a flexible member 4t may be provided in the insert forming all or part of the male connecting member 32. Referring to Figs 11A and 11C, a U-shaped slot or any suitable separation or reduction of thickness may be provided in the male connecting member 32 to separate the flexible member 4t from a remainder part of the insert, which may be an inserting tab 4, 104. A flexible member 104t is also provided similarly in the insert of the complementary connector, as reflected in Fig. 11B.

The flexible member 4t, 104t may be provided with a radial protruding lug 40. The radial protruding lug forms a retaining rim to obtain an axial abutment, when engaged against a latching surface or similar latching means LM, when the lug 40 extends beyond the axial access 30 in the female connecting member 31 (in connected state). For the mechanical coupling, each inserting tab 4, 104 may have a free end engaged/received in a groove G8 (Figs 11B-11C) arranged in the housing 8, while the flexible member 4t, 104t (possibly separated from the free end by a slot or thinning portion) is engaged at a rear of the latching means LM included in a rim portion of the flange 3. Like the case of a locking slider 10:
- position of the latching means LM may be intermediate between the external side wall 18 and the intermediate wall FW,
- and/or a sliding displacement of the external actuator 12 (when not blocked by the actuation blocker 14, i.e. with provision the plug 15 is in a sealing position) allow disengagement between the flexible member 4t and the latching means LM, for instance by having the abutment member sliding in a slide of the housing 8 to push the lug 40 or similar retaining relief of the flexible member 4t, preferably with a centripetal pushing, thus causing a flexing of the flexible member 4t that is no more facing the retaining surface of the latching means LM.

In these illustrated embodiments, the inserts (first and second inserts) can be disengaged simultaneously, typically with the fastening regions formed by the flexible members 4t, 104t released from their respective latching means (two latching means LM are provided in the connection system). It is understood that use of four connecting members (with synchronized interaction for the two pairs of interlocked members) makes the connection robust.

### Fluid Coupling using the two connectors

Referring to Figs 1-2 and 4A-4C, the fluid coupling components A and B can be identical, each provided in a connector 1, 101 of similar or identical structure. Each axial passage/access 30 may extend on a side of the opening O, which is opposite relative to the side where the inserting tab 4, 104 or similar insert extends. The opening O may be a central opening, so that it may provide an axial passage, through the flange 3 and extended by the tubular body 2. This axial passage is rotationally symmetrical about the longitudinal axis X, allowing passage of the fluid conveyed through the fluidic connection.

The coupling ends or components A, B may be identical, each with the same female connecting member 31 and the same male connecting member 32, so that the connectors 1, 101 are genderless. Practically, the connectors 1, 101 such as illustrated in Figs 4A and 6 may be identical assemblies, with the connector 101 positioned in the reverse direction and rotated 180° as compared to the connector 1. While Fig. 4A shows the coupling before removal of the membranes M, Fig. 6 illustrates an operational configuration, in which the connector 1 shown on the left has been set in a closing state, with the position P2 well apparent for a lug 15 that is carried by the plug 15 of the connector 1.

Since synchronization means MP are formed at the interspace (within the gap e), and more particularly between side covering portions 119 of the external actuators 12, disconnection is easy, provided the two sleeves 20 have been properly commanded to drive the plugs 15 (each in closing position). The external actuators 12 may have each a pair of side covering portion 119, covering the external side wall at three sides, with a retaining relief R1 engaging open ends 12b, 12c of the arms 12a, to allow a retaining of the external actuators 12. The opening 012 may be provided at apposition distal from the pushing surface PS. A rim B12 cooperating with the retaining reliefs R1 can prevent extraction of the external actuator 12 once this latter is clipped on the flange 3.

### Non-limiting exemplary use

In some embodiments, the connection sequence for mating the fluid connector 1 to a second compatible aseptic connector 101 is as follows. First, the fluid connector 1 starts with its valve plug 15 in its open position but with the membrane M sealing the opening O. Then, using the aseptic attachment structure 5, an aseptic connection is made between the aseptic fluid connector 1 and another compatible aseptic fluid connector 101, which may be possibly identical (situation where the connector 1 is genderless). Two membranes M are extending parallel and in mutual contact, before a combined removal of these membranes M. While hose nozzle with at least one barb 36 or similar connecting male member is provided at the rear end of each connector 1, 101 or at a rear of the fitting RC in the drawings, it is understood than any other connecting part may be used, possibly with the connector directly welded on a pouch, a bioreactor or any device used in a circuit where a biopharmaceutical fluid can flow.
Each actuation blocker 14 is kept in its initial position, preventing any displacement of the external actuator 12, which means no unlocking action can be made. The passage of fluid, after removal of the membranes M, can then be controlled by handling at least one of the sleeves 20. Mechanical disconnection for spacing the connectors 1 and 101 is available only after handling the two sleeves 20, optionally without any possibility of re-opening the fluid passage if the lugs 15f or similar plug parts are retained in a definitive position reflecting end of the fluid transfer/circulation operation.

Each actuation blocker 14 is manually displaceable when moving the sleeve 20 and a non-blocking position is selectively obtained when the sleeve 20 has been rotated to drive the valve plug 15 in its plugging position, which may imply grasping the external actuators 12 that remain fixed, due to:
- the current blocking position of the actuation blocker;
- and the fact that already releasing an external actuator 12 is not sufficient, as the synchronization means are preventing a displacement of a sole external actuator without the associated/coupled external actuator of the other connector.

Whatever the precise structure chosen for the sleeve actuation, possibly with a retaining effect to prevent reverse rotation of the sleeve 20 once the sealing position is obtained, the connector is of interest for single-use application. From a control point of view for a "single use" system, the connector 1, 101 is compliant with starting a connection, which is aseptic, while also allowing aseptic disconnection since no open end communicates with the fluid present in the interior space 7. Optionally, mechanical connections and disconnections are still available at the flanges 3 of the connectors 1, 101, but the interior space 7 remains closed: no fluid passage can be re-opened in preferred embodiments.

Any reference sign in the following claims should not be construed as limiting the claim. It will be obvious that the use of the verb "to comprise" and its conjugations does not exclude the presence of any other elements besides those defined in any claim. The word "a" or "an" preceding an element does not exclude the presence of a plurality of such elements.

## Claims

1. A connector (1) for fluid connection, comprising a valve assembly (VA) that is provided with a tubular body (2), a plug (15) movable in an interior space (7) of the tubular body (2) and a sleeve (20) to control position of the plug inside the tubular body (2), the connector (1) comprising:
- a flange (3) formed peripherally on the tubular body (2), which extends around a longitudinal axis (X), the flange (3) comprising a flange front face and a female connecting member (31) with an axial access (30);
- a male connecting member (32) including a first insert protruding axially along a protruding direction, the first insert including a fastening region (4r; 4t);
- an aseptic attachment structure (5), including a connector front face and a membrane (M) that is removably attached to the connector front face to seal an opening (O) of the tubular body (2), the connector front face preferably including the flange front face;
- a flange peripheral region extending around the interior space (7), preferably at a rear of the flange front face to delimit a recess (R8) opening rearwardly, the axial access (30) forming an access to the flange peripheral region and allowing insertion of a second insert of same structure as the first insert, the second insert including a fastening region (104r; 104t), the second insert being formed in a complementary connector (101) that is suitable to be coupled to said connector (1) for enabling the fluid connection;
- latching means (LM) configured to allow the second insert, arranged in the flange peripheral region, to be axially retained in a locking configuration of the fluid connection by an abutment at the fastening region (104r; 104t) of the second insert, the locking configuration being obtained when the second insert is inserted in the female connecting member (31);
wherein the connector (1) is configured to allow a disconnection, by releasing the fastening region (104r; 104t) of the second insert from the latching means (LM), after successively:
- moving the plug (15) in a closing position by a first manual actuation using the sleeve (20); and
- unlocking the locking configuration of the second insert by a second manual actuation using an external actuator (12) mounted on the flange (3), preferably covering the flange (3), the second external actuator (12) being unblocked due to the first actuation with the sleeve (20) moving an actuation blocker (14) preventing the second manual actuation.

2. The connector according to claim 1, wherein the tubular body (2) extends around the longitudinal axis (X) between a first open end (2a) and a second open end (2b), the flange (3) being integral with the tubular body (2)
wherein the aseptic attachment structure (5) includes the second open end (2b), the membrane (M) being removably attached to the flange front face (F3) or the tubular body (2) to seal the opening (O) provided at the second open end (2b), the interior space (7) of the tubular body (2) being accessible via the opening (O);
wherein the flange peripheral region is under form of a housing (8) delimited by an external side wall (18) of the flange (3);
wherein the plug (15) is movable in the interior space (7) and has a sealing position inside the interior space (7) when reaching the closing position,
wherein the external actuator (12) is operatively coupled to the latching means (LM) so that a displacement of the external actuator (12) causes release of the engagement, which is an abutment engagement between the latching means (LM) and the second insert, whereby the external actuator (12) can unlock the fluid connection;
wherein the actuation blocker (14) is configured to prevent the displacement in a first actuation blocker position, which is a position in a first angular sector around the tubular body (2);
and wherein the plug (15) is coupled to the sleeve (20) and guided by the tubular body (2), the sleeve (20) extending around the tubular body (2), the actuation blocker (14) having a second actuation blocker position;
and wherein the second actuation blocker position, which is obtained in response to an actuation of the sleeve (20) to have the actuation blocker positioned in a second angular sector around the tubular body (2), allows the displacement.

3. The connector according to claim 2, wherein the plug (15) is movable between a distal position away from the second open end (2b) and allowing a fluid to flow through the second open end, and a proximal position in which the second open end (2b) is engaged by the plug (15) to seal the opening (O) and allow an aseptic disconnection of the connector (1).

4. The connector according to claim 2 or 3, wherein the sleeve (20) has a first position, in which the actuation blocker (14) is facing an abutment part (12d) of the external actuator (12) and prevents said displacement of the external actuator, the sleeve (20) being rotatably movable to occupy a second position in which the actuation blocker (14) is offset relative to the abutment part (12d), the second actuation blocker position being selectively obtained for the second position of the sleeve;
and wherein the sleeve (20) is selectively arranged in the first position to maintain the plug (15) in the distal position, so that unlocking of the fluid connection by the displacement of the external actuator (12) previously requires moving the sleeve (8) in the second position.

5. The connector according to any one of the preceding claims, wherein the flange (3) comprises the male connecting member (32), the first insert protruding axially from the flange front face (F3) provided with the axial access (30).

6. The connector according to claim 5, wherein the male connecting member (32) and the female connecting member (31) are distributed on the flange front face (F3), so that the connector (1) is genderless.

7. The connector according to any one of the preceding claims, wherein the insert comprises an inserting tab (4) that is offset on a first side relative to the longitudinal axis (X), the latching means (LM) being radially movable, preferably toward the longitudinal axis (X), to engage the fastening region (4r)

8. The connector according to claim 7 combined with claim 6, wherein the inserting tab (4), which protrudes from the flange front face, is offset on a first side relative to the opening (O), while the female connecting member (31) is offset on a second side, opposite to the first side, relative to the opening (O),
and wherein a base of the inserting tab (4), the opening (O) and the axial access (30) of the female connecting member (31) are in alignment along a transverse direction perpendicular to the longitudinal axis (X).

9. The connector according to any one of the preceding claims, wherein the sleeve (20) and at least one protruding part (15f) protruding radially outward, provided on the plug (15), cooperate in a bayonet connection system.

10. The connector according to claim 9 when depending on claim 4, wherein the sleeve (20) comprises:
- a guiding track (GT) to guide movement of the at least one protruding part (15f);
- a retaining element (2d) arranged at an end of the guiding track (GT) that is proximal relative to the flange (3),
wherein the plug (15) is in the proximal position when the at least one protruding part (15f) is engaging with the retaining element (2d).

11. The connector according to any one of the preceding claims, wherein the external actuator (12) is covering three distinct sides of the flange (3) and is slidably mounted relative to the flange, the external actuator (12) comprising two sliding arms between which the female connecting member (31) and the male connecting member (32) extend.

12. The connector according to any one of the preceding claims, wherein the flange peripheral region is under form of a housing (8), the housing (8) forming a slide (80) extending generally within a transverse plane (YZ) perpendicular to the longitudinal axis (X),
wherein the latching means (LM) comprise a locking slider (10) distinct from the tubular body (2) and inserted in the housing (8), the locking slider (10) being configured to perform the retaining of the second insert (104) at the fastening region (104r) of the second insert (104).

13. The connector according to claim 12, wherein the external actuator (12) is pushable along a first direction (Z) and the locking slider (10) is configured to provide an elastic return force directed toward a second direction (Y) to radially engage the insert, the second direction (Y) being substantially perpendicular to the first direction (Z).

14. The connector according to claim 13, wherein the external actuator (12) is provided with an abutment member (R12),
and wherein a slanted surface is provided in at least one amongst the abutment member (R12) and the locking slider (10), the slanted surface being configured so that movement of the external actuator (12) along the first direction (Z) causes the locking slider (10) to be driven along an unlocking direction that is opposite to the second direction (Y).

15. The connector according to claim 14, wherein the flange peripheral region is under form of a housing (8) provided with an external side wall (18) of the flange that delimits a recess (R8),
wherein the external actuator (12) is pushable along a first direction (Z) and is provided with an abutment member (R12) to drive one amongst the latching means (LM) and the fastening region (104r) via an opening (018) provided in the external side wall (18),
and wherein the abutment member (R12) is adjacent to the abutment part (12d) and/or protrudes inside the recess (R8).

16. The connector according to claim 15, wherein the recess (R8) is of annular shape for receiving the latching means (LM) in the recess (R8),
wherein the abutment member (R12) is configured to drive the latching means (LM) via the opening (018),
and wherein the abutment member (R12) radially protrudes inside the recess (R8) at a corner portion of the housing (8).

17. The connector according to any one of the preceding claims, wherein the valve assembly (VA) is springless, the sleeve (20) comprising at least one cam slot, in which one or more radial protrusions of the plug (15) engage,
wherein the tubular body (2) has a rectilinear slot (2f) interposed between the plug (15) and the sleeve (20), the one or more radial protrusions protruding radially through the rectilinear slot (2f).

18. A connection system comprising a first connector (1) and a second connector (101), which are each a connector for fluid connection as defined in any one of the preceding claims, wherein a difference in angular position between the first connector (1) and the second connector (101) is 180° in an operating mode of the connection system with the respective flanges of the first and second connector being facing mutually, with an annular contact provided between two annular seals (J), each of the two annular seals (J) being carried in the corresponding connector (1, 101) on a flange face, so-called flange front face, that is opposite to a recess (R8) of the peripheral region, in which the latching means (LM) extend,
and wherein, when the plugs (15) are in their sealing position, the external actuator (12) in the first connector (1), on the one hand, and the external actuator (12) in the second connector (101), on the other hand, are simultaneously pushable along a same direction, due to a longitudinally extending interconnection (33, 33a) linking the external connector of the first connector (1) to the external connector of the second connector (101).

19. A method of assembling a fluid connector (1), the method comprising:
- inserting a plug (15) inside a tubular body (2) via a first open end (2a) of the tubular body, which is a tubular body rear end;
- coupling a sleeve (20) to the plug (15) while leaving a radial interspace between the sleeve (20) and the plug (15), so that a guiding portion of the tubular body (2) can extend in the radial interspace;
- covering a second open end (2b) of the tubular body, which is a tubular body front end, by a membrane (M), in order to seal a central opening (O) of the tubular body (2);
- forming an aseptic attachment structure (5) with a flange (3) formed peripherally on the tubular body (2), in order to form a flange front face (F3) partly covered by the membrane (M) while a male connecting member (32) offset relative to the membrane (M) and arranged integral with the tubular body (2) is protruding frontward beyond the membrane, the flange (3) being provided with a female connecting member (31) having an axial access (30) and preferably with the male connecting member (32) that protrudes from the flange front face (F3), the membrane (M) extending radially, preferably beyond the flange to provide a pull portion, along a first transverse direction (Z) that is perpendicular to a longitudinal axis (X) of the tubular body passing through the central opening (O);
- housing a latch (LM) or a retaining surface in a recess (R8) formed by the flange (3), the axial access (30) axially opening in the recess (R8);
- mounting an external actuator (12) on the flange (3), so that the external actuator (12) can be pushed along the first transverse direction (Z) and/or pushed from a pushing surface (PS) of the external actuator (12) which extends along a second transverse direction (Y) that is transverse or perpendicular to the first transverse direction (Z);
- engaging the latch (LM) or the retaining surface in the recess (R8) to provide a return-effect or clipping allowing the latch (LM) or the retaining surface to interfere with a crossing insert (104) of another connector, which is an insert of same length and shape as an insert (4) provided in the male connecting member (32), the crossing insert (104) passing through the axial access (30) and protruding in the recess (R8) to be automatically engaged by the latch (LM) or the retaining surface;
- placing an actuation blocker (14) in an interposed position between the pushing surface (PS) and the sleeve (20), which is a blocking position so that the actuation blocker (14) prevents the external actuator (12) to be moved radially inward when pushed along the first transverse direction (Z);
whereby the latch (LM) or the retaining surface can retain the fitting insert (104), in a locking configuration established by interconnecting the connector (1) with another identical connector,
wherein in mounted state, the external actuator (12) is actuatable and movable to have an engaged state, in which the external actuator (12) engages one amongst:
- the latch (LM) or the retaining surface,
- and the fitting insert (104),
the engaged state being obtained in a pushed state of the external actuator to unlock the retaining of the fitting insert (104), provided the actuation blocker (14) has been previously removed from the blocking position;
and wherein the sleeve (20) is configured to be manually operable to simultaneously:
- move the plug (15) to reach the tubular body front end in a closing position sealing the central opening (O), in absence of the membrane (M); and
- remove the actuation blocker (14) from the blocking position.

20. Use of two connectors (1, 101) for fluid connection that are each as defined in any one of claim 1-17, wherein the two connectors (1, 101), genderless, are mutually fastened at the flanges (3) that are facing one each other with an interspace (e), each flange (3) carrying the external actuator (12) that is movable relative to said flange (3) to allow a release of a retaining contact at the latching means (LM),
and wherein each connector (1, 101) has the external actuator (12) comprising a front portion that is:
- partly covering the flange (3) that supports the associated external actuator (12),;
- facing in a direction away from the sleeve (20); and
- provided with a linking member or pin (33, 33a) that protrudes from the front portion;
so that in a connection system obtained by coupling the two connectors, a link is obtained by the bridging members or pins (33, 33a) so that a disconnection of the fluid connection system is only available when having:
- each actuation blocker (14) of the respective connectors (1, 101) occupying the second actuation blocker position, which means each plug (15) is in the sealing position in the two connectors (1, 101).
